# EUROPEAN PATENT APPLICATION

(11) **EP 1 839 701 A1**
(43) Date of publication of application: **03.10.2007**
(21) Application number: 07251320.3
(22) Date of filing: 28.03.2007
(51) Int. Cl.: A61N 1/372, A61F 2/02, G06F 19/00, A61B 5/00

(54) **Embedded neural prosthesis**

(30) Priority: 31.03.2006 US 394520
(71) Applicant: Cordis Corporation, Miami Lakes, FL 33014 (US)
(72) Inventor: Lorincz, Andras, 2094 Nagykovacsi (HU); Meretei, Attila, Pompano Beach, FL 33062 (US); Palotai, Zsolt, 212 Veresegyhaz (HU)
(74) Representative: Belcher, Simon James

(57) **Abstract**

A neural prosthesis comprised of a network of component devices that sense, analyze, and communicate data to deliver a therapeutic effect to a patient. The network of component devices establish systems and methods for sensing physiological parameters in, on or around a human body and achieving a therapeutic effect based thereon. A network of various levels of component devices sense, process and communicate data between corresponding component devices, and self-organize into a hierarchy of peer groups of component devices to perform the task or function of the therapeutic effect upon completion of the tasks or functions of the various underlying levels of component devices. An overall Peer Group encompasses the various underlying levels of peer groups having the component devices therein. The sensing, computational, data distribution, communication or therapeutic effect tasks at the various levels are accomplished by the coordination of communication and functions between the plurality of relatively simple component devices of the network. Symmetric and asymmetric cryptography and other communication protocols are used to co-ordinate the tasks and functions of the component devices of the network. Therapeutic tasks such as drug delivery, executable actions, and stimuli delivery or suppression are thus efficiently distributed to a patient via the network. Component peer devices of the network can be implants, wearable devices with respect to a patient, or may be devices that are in the environment within which the patient is located.

## Description

The invention generally relates to medical devices that repair, replace or restore lost neural function. An embedded neural prosthesis provides a network architecture for repairing, replacing or restoring lost neural functions. More specifically, the embedded neural prosthesis provides systems and methods for sensing physiologic parameters and achieving a therapeutic effect in the nervous system by using a multitude of devices that dynamically self-organize into a network of devices. The network devices communicate with one another to adjust the function of various individual devices in order to optimize the overall function of the network of devices. According to the systems and methods of the embedded neural prosthesis, the individual devices may be implanted in or applied onto the body of a patient, may be in an environment external to the patient, or may be some combination thereof.

Neural prostheses are medical devices that restore lost or damaged neural function. These devices are usually implants. A neural prosthesis typically relies on sensing, information processing, and communication with the nervous system or other devices to perform its functions. An embedded neural prosthesis is a medical device that is connected to the nervous system and is operated by information received from a multitude of sensory and computing devices in its environment.

Sensing of physiological conditions occurring within the human body or other conditions occurring in the environment within which the human body is located, and co-ordinating therapeutic interaction with the human body in accordance with the sensory information obtained is generally accomplished by complex medical devices. Such medical devices typically have built in sensing, computation, communication and additional modules that are responsible for determining and delivering an appropriate therapeutic response based on the sensory information obtained. For example, an implantable cardioverter defibrillator device will contain all of these functions within one device in order to obtain information regarding cardiac activity in a patient and deliver an appropriate response to encourage ideally normal cardiac activity in the patient.

In some cases, the various functions of such medical devices may be divided between distinct devices. For example, a sensor may be implanted in one part of the body to conduct sensing and communication functions, and a therapeutic device may be implanted in another part of the body to perform communication, computation and therapeutic functions. In this case, the sensing unit would likely measure certain physical, chemical or biological parameters of the body, and transmit this information to the therapeutic unit, whereas the therapeutic unit would analyze the data received from the sensing unit and initiate therapeutic action according to the outcome of the data analysis conducted in the therapeutic unit. Alternatively, computation and data analysis may be performed by the sensory unit.

In each of the above cases, the medical devices are generally very complex. This complexity increases the likelihood of failure of the device. Such a complex medical device, for example, may fail in any of the sensing, computing, communication or therapeutic action modes. A failure of any one of these modes may lead to a critical loss of functionality, and the loss of therapeutic action, which in turn may endanger the health of the patient. This is especially an issue in those cases where only one device is responsible for maintaining a sufficient therapeutic effect. Loss of function of a single device may thus have a major negative impact on the health of the patient, even risking death in some instances.

Furthermore, the therapeutic effect of many devices is limited to the range of minimum and maximum values of therapy a single device is able to deliver. Thus, although the therapeutic effect that can be delivered from a single device may be adjusted between the extreme (minimum and maximum) values, for example, the maximum value cannot be exceeded even if that becomes necessary as a sensing unit may determine. The device in that case has to be replaced by another device that has a higher maximum capacity. This replacement requires a visit to the health care provider at a minimum, and may also require an invasive intervention in which the low capacity medical device is removed and a high capacity device is implanted.

A further limitation of single devices is that their sensing and therapeutic functions may be localized. For example, such a device may measure a physiological or other parameter in one location of the patient and deliver a drug into one specific blood vessel in response. While this may be effective where localized conditions merit localized treatments, there may be occasions when sensing parameters in a multitude of locations, as well as delivery of a therapeutic effect in a multitude of locations, is desired. In light of all these observations, a need exists for more robust medical devices that are constructed for an even higher degree of functionality than current complex devices. Such more robust medical devices are comprised of a multitude of devices comprising a network of devices, each device having a simpler function that, when networked with other devices, provides more complex functions than could be accomplished individually or by prior medical devices. The more robust medical devices would thus ideally provide sensing and therapeutic functions in local or multiple locations in a patient simultaneously such that a wider range of therapeutic effect adaptable to actual needs is accommodated.

Along these lines an embedded neural prosthesis comprises a multitude of component devices. The task of sensing may be accomplished by multiple distributed and networked sensors that monitor physiological data and environmental factors, and help determine the therapeutic effect to be delivered from some level of devices in the network of devices in response to those factors. These sensory component devices are networked and may perform distributed computing on the data they acquire from the patient or the environment and then send instructions to therapeutic component devices of the same embedded neural prosthesis. The therapeutic component devices may then alter neural function by delivering stimulation, eliminating or masking certain natural signals, administering drugs, or the like.

The neural prosthesis in this case is embedded in its own ambient intelligence system of sensory and computing component devices that influences the operation of its therapeutic subunits.

The systems and methods of the invention use a multitude of devices that are implanted in the body of a patient, attached externally to the body of the patient, or located in the environment within which the patient is located, or some combination thereof. The multitude of devices is able to self-organize into a dynamic network of devices to perform individual and collective functions once positioned as desired relative to the patient. At a minimum, each device communicates with at least one other device within range of one another. Each device may also communicate with other devices within its range, compute and store data, distribute data, deliver a therapeutic effect and, in some cases, communicate among multiple devices beyond its range using appropriate communication protocols.

Each component device, also referred to as "device" or "peer device" herein, after it is positioned as desired in, on or around the patient, establishes a communication link with other component devices located within its communication range. Some devices in the network are directly connected to each other and can exchange data directly. Other devices in the network are not directly connected and exchange data, or otherwise communicate, indirectly by message hopping. Message hopping means that message are sent and received through a chain of intermediary component devices, or by pipeline operations, using appropriate communication protocols. All component devices that are linked by either direct or indirect communication protocols can pass messages to and from each other through various component devices that belong to the same network or array of devices.

Each component device may have a single function or a combination of multiple functions. For example, one component device may primarily act as a sensory unit in the network and may have no therapeutic effect at all. Such a sensory unit would be primarily responsible for measuring a physical, chemical, biological or other physiological parameter within range of the sensory unit in, on or about the patient's body. This sensory unit would then communicate the measurement data obtained to other component devices in the network that would then process this data and, where appropriate, initiate the function of other component devices. Another component device may have a combination of a sensory function and a computing function, and may have the ability to perform some processing of raw sensory data locally. Other combinations of functions onboard a component device are also conceivable. However, each component device preferably has at least an elementary communication function, (e.g. the ability to send or receive commands) within the network or array of component devices. Moreover, the network of devices comprising the medical device preferably provides at least a therapeutic effect, such as mechanical assistance, actuation, drug delivery, electrical stimulation, or the like, to help repair, replace or restore lost neural function to benefit the patient.

The communication links between component devices within the network help propagate data between component devices within the network and help control the functions of the various component devices according to the sensed physiological parameters of the patient or the environment within which the patient is situated. This data propagation allows the allocation of various tasks among component devices. Task allocation among the various devices makes it possible for the network of devices to perform complex computational, communication, energy management, therapeutic, or other functions, even if the functional capability of individual devices would not allow such complexity. For example, by allocating computational tasks among a sufficiently large number of component devices, a complex task may be accomplished even if the onboard computing power of each individual component device is greatly limited. Similarly, a large therapeutic effect may be achieved (e.g. a sufficiently large dose of drug may be delivered) even if the therapeutic capability of individual devices (e.g. the amount of drug available for release from one device) is limited.

The allocation of tasks among the devices of the network is a dynamic process in order to accommodate the changing conditions and physiological parameters of a patient or the environment within which the patient is situated. Should certain component devices lose complete or partial function, be destroyed or removed from the network, or should new devices be introduced into the network, then task allocation among currently operational devices adapts to the new network configuration, and to the actual availability of resources within the network, by self-organizing the component devices of the network to achieve intended tasks, sub-tasks, etc. in a timely and efficient manner. The self-organizing approach of component devices within the network, as described herein, increases the adaptability of the overall medical device in terms of sensing, computing, communicating data between component devices, and delivering of therapeutic functions to the benefit of the patient.

The allocation of tasks, sub-tasks, etc., among component devices are preferably guided by communication protocols that ensure task allocation is optimized for the efficiency of the network in view of the array of simple component devices comprising such network. Communication protocols are thus provided that allow directed communication between component devices to the exclusion of other devices, in some instances. For example, one component device may direct a message to a specific component device, within its communication range, rather than having to broadcast every message throughout the entire network in order to address an intended other peer device. The inefficiencies of such a network-wide broadcast protocol are self-evident and are minimized according to the peer-specific communication protocols of the systems and methods of the invention. Such peer-specific communication protocols preferably use virtual identifiers for each component device, which emphasizes the need for anonymity and accountability requirements.

Thus, component devices comprising the network of devices of the overall medical device according to the systems and methods of the invention preferably also assemble communication pipelines within the network and communicate the allocation of individual tasks to respective ones of the various component devices. In addition, component devices may route messages to other component devices out of their communication range. Routing of messages from a component device to one or more component devices beyond the communication range of the originating component device can occur through a chain of intermediate component devices. The network is therefore not flooded with messages in order to ensure that a message will arrive at the intended recipient component device. The component devices also preferably perform local communication scheduling, which ensures that collision-free direct communication between intended component devices that use the same communication links is possible. To this end, communication links between devices are preferably assigned such that the recipient component device listens on the same channel as used by the sender component device that sends the message. Further, communication of component devices using the same channel should preferably be scheduled to minimize the probability of message collisions, that is, the sending of a message through the same channel at the same time by more than one component device. The component devices thus also preferably perform task allocation and scheduling to ensure that tasks are allocated to appropriate component devices and each component device schedules its tasks so that intended tasks are accomplished by the fewest possible resources in accordance with quality of service requirements and risk estimations. Component devices also preferably allocate and schedule tasks to minimize energy consumption and the use of critical resources, thereby reducing the overall "cost" of operating the medical device according to the systems and methods of the invention.

The network of component devices, thus generally comprises a hierarchy of various levels of peer groups of devices, the various levels of peer groups comprising an overall Peer Group that in turn comprises the medical device according to the systems and methods of the invention. Each peer group level is assigned a task or a function to perform. The component devices, i.e., peer devices, within a peer group may be further formed into sub-peer groups comprised of sub-peer devices that solve sub-tasks or sub-functions to more efficiently perform the overall Peer Group's task or function eventually. The sub-peer devices in a sub-peer group may further still form sub-sub-peer groups having sub-sub-peer devices that perform sub-sub-tasks or sub-sub-functions, and so on, in order to eventually perform the intended task or function of the overall Peer Group most effectively. The various levels of peer groups are thus logical groups of peer devices that may be created in, on or about a patient in order to sense, communicate, compute and distribute data, and deliver a therapeutic effect based on such data. As the artisan should appreciate, reference to Peer Group denotes the overall Peer Group of the medical device, whereas reference to peer group, sub-peer group, or sub-sub-peer group, etc., is understood to correspond to the respective levels of devices, sub-devices, or sub-sub-devices, etc., for performing the respective tasks or functions, sub-tasks or sub-functions, or sub-sub-tasks or sub-sub-functions, etc., associated therewith, within the context of the medical device described herein even where the various levels are not specifically repetitively referred to herein.

Peer devices in the same peer group need not be physically close to each other, and need not have similar capabilities (like sensing, therapeutic effect, etc.), although it is usually preferable to have at least a chain of peer devices within a peer group through which tasks or functions can be communicated between peer devices of the peer group. Also it is usually preferable to have more than one peer device with a given capability in a peer group for each required task or function of the peer group. Here task and function can be arbitrary actions, like communicating data from one peer device to another, performing some specific computation on the data, sensing some parameters, or bringing about some therapeutic effect, etc. The peer devices of a peer group, or sub-peer devices of a sub-peer group and so on, may be placed in, on, or about a patient in order to conduct the various tasks or functions detailed herein. Execution of an overall Peer Group's task may commence once all of the various underlying tasks or sub-tasks, etc., are accepted and performed by appropriately corresponding peer devices, sub-peer devices, etc. of the various levels of peer groups within the overall Peer Group. The comprehensive allocation of the various tasks or functions before execution of the task or function of the overall Peer Group ensures that at least a minimum level of confidence and reliability between the various levels of peer groups and devices within the Peer Group are achieved before the eventual execution of the Peer Group's task or function. The comprehensive allocation of tasks or functions before execution of any given peer group's task or function also increases the efficiency at which each peer group will execute the various tasks or functions allocated thereto by sequencing the various tasks among devices that have the capacity and resources to execute assigned tasks or functions efficiently. Thus, once a peer group's task or function is ready for execution, the peer devices, or sub-peer devices, etc., can start to work on their assigned task or function, etc. by receiving and processing data according to an optimized and predetermined sequence and schedule to achieve the intended therapeutic effect. Each device thus contains data, algorithms and/or protocols that enable the devices to process some or all of the data distributed within the network, to exchange, modify or reconfigure some or all of the data, and to autonomously allocate data storage, computational, communication, energy supply, timing, sensory and/or therapeutic effect delivery from the various devices comprising the network.

The tasks, sub-tasks, functions, etc., of the various levels of devices can be one time tasks or functions, which are rare, or can be repeatedly executable tasks or functions with a given restart time. In the former case, the task or function can be solved by a single chain of peers in which each peer trusts its successor peer. One device's failure would break the chain, however, thus stopping execution of the task or function until the chain is restored. In the latter case, on the other hand, a single chain of peer devices may be insufficient to perform the intended task or function within the time allotted before restart of the task or function is to occur. Pipeline communication between component devices can overcome this deficiency, however, by permitting continued execution of the intended task or function by downstream component devices while restarting execution of the same task or function by upstream component devices. This latter situation can occur, for example, where a computed amount of drug is to be delivered every 1ms by some peer devices based on sensory information sensed by some peer devices in every 1 ms. However, the computation of the amount of drug to be delivered takes more than 1 ms based on the sensory information obtained. In this case, the task is called a pipeline task, performed best by a pipeline operation that permits the current task or function to be executed even as another similar task or function is initiated.

Survivable Pipeline Protocols (SPP) help to achieve such pipeline tasks or operations by providing a framework that organizes and maintains the various levels of peer groups to execute such pipeline tasks without having a central coordinator in the network of devices. SPP thus enables peer groups to adapt to changes in peer device availability occurring in, on or about the patient where the task is executed, for example, such as when an existing peer device fails or a new peer device is introduced to the network. SPP protocols thus provide a framework for adding, removing and, or reorganizing the peer group of devices without a central coordinator in the network of peer devices. In this way the network of peer devices, etc., continuously adapts to changes in the availability, performance and reliability of various levels of peer devices, etc., to the availability of newly introduced peer devices, or to changes in the task's requirements, such as processing speed, the sequence of performance of tasks, the amount of therapeutic effect, etc., without relying on a central coordinator device. The introduction of a central coordinator device would make the network vulnerable and less apt for survival, as loss of function of the central coordinator may lead to disorganization and loss of function of the entire network.

Generally, an overall Peer Group's task is completed when all of the underlying tasks or functions, or sub-tasks or sub-functions, etc. of the various levels of peer groups within the overall Peer Group are executed. After execution of a task or function, the peer device, etc., involved in such execution can update the relationships among the other peer devices. Of course, upon completion of the executed task or function, the same updating of relationships between sub-peer devices, etc, is likewise performed.

Ideally, component devices are equipped to assess the risk associated with allocating a task or function, or part thereof, to any of the peer devices. This, in turn, allows a component device to select a set of other component devices that are most likely to successfully complete the given task or function, and to direct task or function related messaging to this subset of peer devices.

Component devices may communicate with each other over insecure media, like wireless channels. However, because the information exchanged between devices may contain private, confidential data, some degree of anonymity and accountability in the communication protocols is preferred, in addition to basic security practices regarding data integrity and confidentiality. Anonymity requires that component devices cannot be identified after sending information (e.g., the information cannot be traced back to the device from which it originated), and that virtual identifiers (e.g. an identifier code included in a communication from a device) cannot be linked or traced back to the device from which it originated either. Anonymity thus provides an additional layer of security for the information being communicated between devices in the network and makes it more difficult to launch a targeted attack on an individual component device in the network. Accountability, on the other hand, requires that component devices that stop functioning according to the operating procedures and rules of the network can be identified and expelled or disconnected from the network without compromising the effectiveness of the remaining component devices of the network. Anonymity and accountability are ultimately mutually exclusive requirements. However, a partial reconciliation of these requirements is also possible and contemplated herein. Anonymity and accountability may thus extend to all components of the network, including component devices, patient and caregivers.

Direct communication between peer devices is based on asymmetric and symmetric cryptography. Asymmetric cryptography is used initially to establish trust and secure communication channels between peers, while symmetric cryptography is used later during data communication. Pretty Good Privacy protocol is an example of asymmetric and symmetric cryptography methods that provide message integrity, confidentiality, authentication, non-repudiation, anonymity, and access control. Another example is Anonymous But Accountable Self Organizing Communities, which extends the previous list with accountability. Numerous other methods based on asymmetric and symmetric cryptography are known as well.

Corresponding levels of peer devices within a common peer group, sub-peer group, etc., thus communicate with one another via cryptographic relationships to establish a web of trust between devices within the network, and use Survivable Pipeline Protocols to establish a hierarchy of self-organized devices at various levels in order to execute and maintain prioritized tasks or functions by the devices within a peer group, and to update the trust relationships between the devices. Thereafter, the overall Peer Group executes its intended task or function, which can be the delivery of one or more drugs, delivery of stimuli, or the like to help replace or restore lost neural function based on the physiological parameters sensed and the data messaging and computation that occurred within the various levels of devices and peer groups of the overall Peer Group. Cryptography and Survivable Pipeline Protocols are thus used to co-ordinate data communications between various levels of self-organizing peer devices within at least one overall Peer Group in the network of the medical device according to the systems and methods of the invention.

A simple protocol which provides some level of anonymity and accountability between devices is Pretty Good Privacy (PGP). PGP is based on a relationship secured by public key-private key cryptography between peer devices, sub-peer devices, etc. A private key authenticates the originating peer device, sub-peer device, etc. from which data is associated, whereas a public key encodes a given peer device with identity data so that the identity of the given peer device can be determined to see if the given peer device owns the required private key authorizing the acceptance of data that is attempting to be communicated to the given peer device.

In PGP the public key of each peer device, sub-peer device, etc., is thus signed by the private key of at least one other peer device, sub-peer device, etc., thereby creating trust between those peer, sub-peer devices, etc. Trust is maintained between these devices therefore until one or more of the public key signatures have expired, or until the peer device, sub-peer device, etc. has inappropriately performed. Moreover, using PGP, an originating peer, sub-peer device, etc., can communicate with another peer device, etc., directly as detailed above, or indirectly if the public key of an intermediary peer device is trusted by the originating peer device and the other peer device even though neither the originating peer device nor the other peer device has a directly trusted public key for one another. Such a web of trust can grow to connect a variety of peer devices, etc., within the network of the medical device such that eventually each peer device, etc., can communicate to each directly or indirectly trusted peer device, etc., in the chain, without any single peer device, etc., having to store information regarding the public or private keys of all of the various peer devices, etc., in the network. A trusted third party as in the Public Key Infrastructure is thus no longer needed.

If a peer device, etc., fails to work according to the rules of the community then the other peer devices, etc., will not sign this malicious peer device's public key after the previous signatures expire. The malicious peer device, etc., will thus no longer be trusted among the peer devices in the web of trust and therefore will be excluded from the network of devices. The term "malicious" means any of several types of malfunctioning of the peer devices. For example, such a malfunctioning peer device may occur when the peer device is temporarily unavailable because the device has other superceding tasks to perform, when the peer device has to recharge its batteries, or simply when the device is out of communication range in the network. If a peer device is malfunctioning, i.e., unavailable for the network, for any of the reasons set forth above, such malfunctioning is automatically detected by the other devices, because the unavailable device does not respond to communication.

Other forms of malfunctioning can also occur in peer devices, which can be more serious in terms of overall network performance. One of the more serious peer device malfunctions occurs when a peer device can no longer suit the basic functional requirements of the community, as when a hardware part of the device has broken down, for example. This latter malicious peer device thus cannot provide data integrity, security or some communication functions. This type of malfunctioning is detected by other devices in the network because the malfunctioning device can not execute some basic functions of the community, such as those used for task execution, communication, etc. The most serious form of malicious, i.e., malfunctioning, peer device tends to be, however, the malfunctioning peer device that intentionally tries to disturb the working of the network of devices. This last type of malicious peer device is best discovered by specific algorithms to help thwart the intentional efforts of the malicious peer device towards the network of various levels of peer devices. Hereafter, the term "malicious" is thus understood to mean any of the types of malfunctioning devices detailed above.

After two peer devices, etc., agree to communicate with each other, i.e., establish trust, a symmetric key may be generated through which future communications between the trusted peer devices can occur. The peer devices then encode and decode data with that symmetrical key relationship, instead of the more cumbersome public-private key relationship. Once established, the symmetric key relationship requires less computation from the peer devices, but guarantees the same or even higher security for the duration of the communication.

The systems and methods of the invention thus provide a medical device for sensing physiological parameters in, on or around a patient and achieving a therapeutic effect to help repair, replace or restore lost neural functions for the patient with a network of hierarchically arranged peer groups comprised of relatively simple component devices that are able to self-organize into a dynamic, collaborative hierarchy to accomplish various levels of tasks or functions. The failure of any one component device therefore does not significantly impact the performance of the network, but rather reduces the functional capability of the entire network of the medical device by a small amount only. Ideally, therefore, the medical device will not experience complete loss of function even when one or some of the component devices comprising the network fail. Each component device can have a relatively inexpensive and simple structure that individually performs simple functions but that collectively, when assembled within the network, is able to contribute to the performance of more complex functions. The small size of the component devices reduces volumetric intrusion of a patient if implanted or attached to the body of the patient. The small size of the component devices also accommodates dispersion of the devices throughout the body or in the environment within with the body is located, in order to sense or achieve a therapeutic effect in multiple locations simultaneously and in concert.

A neural prosthesis may be constructed based on the principles described in the preceding paragraphs. Such a neural prosthesis performs a vital function by replacing, reinforcing or repairing a neural function. The replacement, reinforcement or repair of neural function is achieved by therapeutic component devices that have the ability to stimulate or depress the function of certain components of the nervous system. To provide such stimulation or other functions to the patient, these therapeutic component devices receive information from multiple sensory and computational devices of the same neural prosthesis. In other words, the operation of the therapeutic devices is embedded in the sensory and computational network of devices. This sensory and computational network of devices helps repair, replace or restore the coupling between the patient and its environment that was otherwise compromised by neural damage.

Both overall therapeutic effects, sensory data collection and computation are distributed among a multitude of component devices, contributing to the robustness, resilience and flexibility of the medical device. A therapeutic device, for example, can also have a role as a sensory unit or as a computing unit. As a minimum, each component device must have a communication capability that enables the component device to act in unison with the rest of the network, in order to effectively act as an integral component within the context of the network, as opposed to as an isolated device.

The artificial coupling between environment and the nervous system of the patient provided by the network of component devices can be further modulated by direct patient, or patient care-giver, input. For example, the patient may be able to indicate that a certain functional status of the network of devices provides more benefit (e.g. better control over the movement of a limb, better quality of replaced hearing or vision, etc.) than another functional status of the network of devices. The embedded neural prosthesis may thus be trained by such patient input. Alternatively, or in addition, patient care-givers may also provide invaluable input directly to the network of devices to help train the embedded neural prosthesis appropriately so as to provide the most beneficial impact to the patient.

As an example for such a distributed embedded neural prosthesis, numerous simple neural implants, i.e., component devices, can be implanted into various portions of a patient's brain and nervous, or other, system and networked with one another to monitor brain activity in one or more areas of the patient's brain. This brain activity monitoring can be further augmented by monitoring other physiological factors, such as body temperature, skin impedance, sweating (moisture), musculo-skeletal movements, breathing patterns, etc. in accord with appropriate component devices strategically placed within, on or near the patient to collect the respectively targeted data. Such data, collected by relatively simple networked sensors, can be processed and communicated to other component devices within the network of devices in order to initiate or modulate the electrical or chemical activity of therapeutic neural implants, i.e., component devices, associated with certain areas of the brain. The activity may be electrical or chemical stimulation, or may be the depression of such electrical or chemical activity, for example, in accord with the data collected. Stimulation or depression delivered by these therapeutic implants is directed by the information collected by the sensory component devices. Computation and analysis of the sensory data may be also distributed between the sensory and therapeutic units, or a computing component device may be introduced into the network.

In general, any component device, in the network of devices comprising the medical device according to the systems and methods described herein, may have all functions, (e.g. sensing, computing, therapeutic effects, etc) or any component device may be specialized to a single function, or just a few functions. In any case, all component devices are enabled to perform some form of communication, i.e., either active communication powered by onboard energy sources, or passive communication that does not require onboard power.

The neural prosthesis described above may be used to treat such diseases as epilepsy, for example, or other neurologic illnesses or conditions. In the case of epilepsy, the sensory network monitors an epileptic patient for signs of an impending attack. Once such signs are detected, the embedded neural prosthesis may send an alert to the patient, physician or caregiver, while also initiating treatment to the patient by activating its therapeutic component devices to alter neural conditions and, ideally, thwart the impending attack before onset of the attack occurs. The therapeutic component devices may utilize chemical, electrical, or other means to alter the neural conditions in the patient in order to thwart or suppress the impending attack, for example.

Embodiments of the invention will now be described by way of example with reference to the accompanying drawings, in which:
Figure 1 illustrates a network comprised of component devices forming at least one Peer Group according to the systems and methods of the invention.
Figure 2 illustrates the direct (in-range), and multi-hop (out-of-range or indirect) communication of component devices.
Figure 3 illustrates two examples when a component device should change its communication range.
Figure 4 illustrates that the devices should communicate through the closest (or lowest) possible peer group, although all of the devices would be able to communicate through the overall peer group PG1.
Figure 5 illustrates generally a direct, cryptographic communication scheme between component devices according to the systems and methods of the invention.
Figure 6 illustrates the propagation of trust among component devices, which enables the establishment of trust between two component devices through a chain of trusted component devices.
Figure 7 illustrates a set of component devices executing a function of a medical device, whereby an exemplary peer group hierarchy created to execute and maintain the function is also shown.
Figures 8a and 8b illustrate a process of ensuring enough "maintaining" devices are available in a peer group and ensuring a "manager" device exists within a peer group to manage communication, data processing and other functional schedules between devices in a peer group.
Figure 9 illustrates how a component device reacts to an advertisement for a task that the network of devices needs to execute.
Figure 10 illustrates how a component device reacts to an advertisement of resource requirement for a sub-task that the network of devices needs to execute.
Figure 11 illustrates how a "manager" device oversees the resourcing and execution of a sub-task within a peer group.
Figure 12 illustrates how a "successor sub-task" is allocated to another component device that becomes responsible for executing the successor sub-task.
Figure 13 illustrates a weblog ranking of component devices according to the systems and methods of the invention.
Figure 14 illustrates a local communication scheme to schedule communications between component devices with minimal interference according to the systems and methods of the invention.
Figure 15 illustrates a routing scheme for communicating data from one component device to another using intermediary component devices according to the systems and methods of the invention.
Figure 16 illustrates one embodiment of a network of component devices for sensing various parameters and delivering a therapeutic effect based thereon according to the systems and methods of the invention.
Figure 17 illustrates exemplary sensing and stimulating electrode arrays implanted in the brain for collaborating with other component devices elsewhere in, on or around the patient to influence the neural activity in the patient according to various systems and methods of the invention.
Figure 18 illustrates a network of component devices designed to receive direct patient or care-giver input and to sense environmental parameters to deliver a therapeutic effect according to the systems and methods of the invention.

The systems and methods of the invention described herein comprise a medical device that is intended to approximate the cellular structure of an anatomical organ or organs, for example, of a living being. The medical device is comprised of two or more relatively simple component devices that self-organize into various levels to hierarchically arrange a network of the component devices. The hierarchical network performs a medically relevant task or function, such as the delivery of a therapeutic effect to a patient, upon completion of tasks or functions associated with the various levels of the hierarchical network. Each device thus contains data, algorithms and/or protocols that enable the devices to process some or all of the data or information sensed, stored and distributed within the network, to exchange, modify or reconfigure some or all of the data, and to autonomously allocate data storage, computational, communication, energy supply, timing, sensory and/or therapeutic effect delivery from the various devices within the network.

Any number of levels of component devices may be provided in the network, although the description herein generally refers to a network having two or three levels in the network. Such levels generally comprise an overall Peer Group, a peer group and sub-peer groups, sub-sub-peer groups, and so on, where desired, and a set of component devices corresponding to each level. Of course, as should be readily evident to the artisan, the network may further be comprised of additional overall Peer Groups and associated levels of peer group, sub-peer groups, sub-sub-peer groups, etc., and component devices, although the non-limiting description herein refers generally to a single overall Peer Group and the various levels associated therewith. A component device may participate in several peer groups simultaneously. Also a component device may contribute to the execution of a task or function at one level of peer group, sub-peer group, sub-sub-peer group, etc., even as another level of peer group, sub-peer group, etc. is established to execute another task or function.

At least one of the component devices is a sensory unit. Where such a network of devices is comprised of predominantly sensory unit component devices, the network will perform primarily a sensory function. The sensory information obtained by the network is then transmitted to other medical devices, the patient, care-giver or other medical personnel. In another embodiment of the network of devices at least one component device is a sensory unit that will primarily perform a sensory function and at least one other of the component devices is a therapeutic effector unit from which an intended therapeutic effect is delivered to the patient in response to data communicated from the sensory unit, throughout the network, and to the therapeutic effector. Additional component devices, other than the sensory unit and the therapeutic effector unit, that perform similar or other tasks or functions may also comprise the network of component devices according to the systems and methods of the invention.

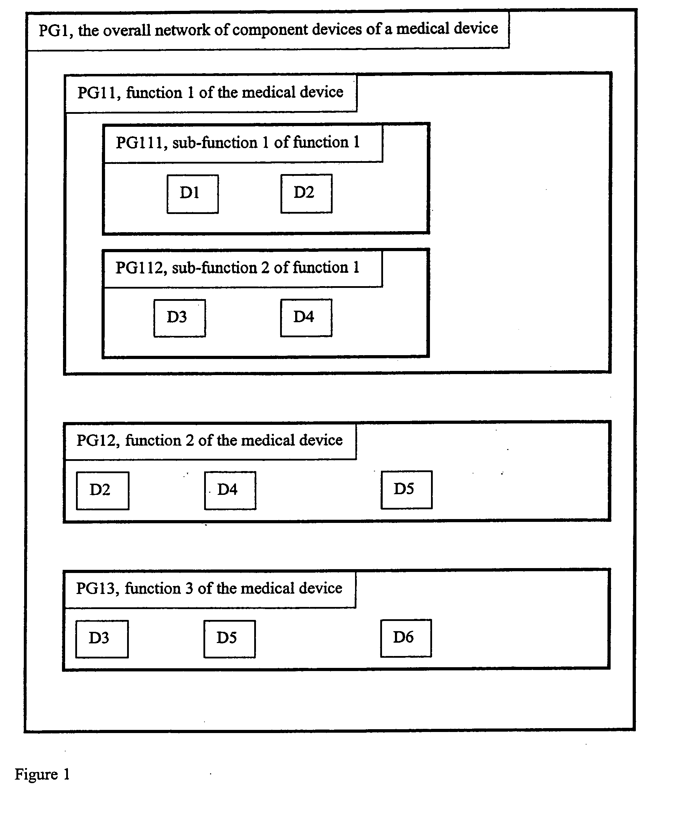

Fig. 1 illustrates schematically a device network forming the overall Peer Group PG1 comprised of peer groups PG11, PG12, and PG13. Specific tasks or functions to be performed by the overall Peer Group PG1 are assigned to the peer groups PG11, PG12, PG13. Each peer group PG11, PG12, PG13 is further comprised of sub-peer groups (like PG111, PG112) and / or component devices (D1-D4; D2, D4, D5; D3, D5, D6, for example). Each component device D1-D6 may belong to more than one peer group PG11, PG12, PG13 within the overall Peer Group PG1 such that each component device D1-D6 may belong to different levels in the hierarchy of the overall Peer Group PG1. Each peer group PG11, PG12, PG13 is assigned a peer group task or function to perform, such as sensing a physiological parameter, computing data or the delivery of a therapeutic effect to a patient. Each component device D1-D6 is assigned a component device task or function to perform within the context of the peer group or sub-peer group with which it is associated, such as sensing a physiological parameter of the patient or the environment within which the patient is located, computing the data, distributing the data, communicating the data to other component devices, or the like. The network of component devices D1-D6, peer groups PG11, PG12, PG13 and sub-peer groups PG111, PG112 thus forms the various levels of the overall Peer Group PG1. The various component devices D1-D6 join some level of the Peer Group, i.e., peer group or sub-peer group as shown, if the component device decides to contribute to the execution of the corresponding task or function associated with the peer group or sub-peer group. If a task or function is too complex to be executed effectively by one component device orpeer group, then the task can be broken up into smaller parts (sub-tasks or sub-functions) and the sub-peer groups (like PG111 and PG112) are created with component devices therein to perform the sub-tasks or sub-functions corresponding thereto. The overall Peer Group PG1, peer groups PG11, PG12, PG13, sub-sub-peer groups PG111, PG112 and component devices D1-D6 are exemplary only, and the artisan should appreciate that a network of devices according to the systems and methods of the invention could comprise any number of at least one overall Peer Group having various levels of peer groups therein comprised of at least two or more component devices. Further, the artisan should readily appreciate that any or all of the various levels of peer groups, i.e., peer groups, sub-peer groups, sub-sub-peer groups, etc., may be further comprised of underlying levels, such as shown in Fig. 1, wherein peer group PG11 is further comprised of sub-peer groups PG111, PG112 for example, and so on. Of course, where provided, each level of peer group would have a corresponding level of task or function to perform.

Generally, higher-level tasks or functions are not performed until all of the underlying levels of tasks or functions are completed. Thus ideally, once each of the underlying peer group level's tasks or functions are completed, the network will have delivered the intended therapeutic effect to the patient based on the physiological parameters sensed, computed and communicated by and between the various levels of component devices and peer groups comprising the overall Peer Group.

Referring again to Fig. 1, each component device D1-D6, for example, performs at least one task or function. At a minimum, therefore, each component device communicates with at least one other component device in the network in order to enable individual devices and the various levels of peer groups within the overall Peer Group to work collectively to perform more complex tasks or functions. Each component device may therefore perform one or more task or function such as sensing physiological or other parameters, computing and storing data, distributing data, and communicating with one or more other devices within the network. Communications between component devices may be one-way to the extent that a component device either receives or transmits data signals or messages but does not do both receiving and transmitting functions, or may be reciprocal, or two-way, such that a component device transmits and receives data signals or messages from at least one other component device.

Populating the patient, the patient's environment, or some combination thereof with component devices enables relatively simple tasks and functions to be performed at various locations in, on or about the patient. Communication links between the component devices help to establish trust and to allocate and prioritize the various tasks or functions among the various levels of component devices within a respective Peer Group of the network. The organization of the component devices within the network thus occurs as a result of the communication links existing between the component devices. The communication between devices may be direct or indirect. Direct communication uses asymmetric or symmetric cryptographic links between trusted component devices, whereas indirect communication uses intermediate component devices with cryptographic links and pipeline protocols. In any case, the communication of simple tasks or functions performed amongst component devices comprising a respective peer group level enables the network of component devices to perform more complex medical functions, such as artificially simulating or controlling organ functions, inducing the control of neural, musculo-skeletal or other organ function by chemical or electrical stimulation, or responsive monitoring based on physiological parameters sensed, manipulated and communicated by component devices of the network. Of course, where more than one overall Peer Group is provided, communication may similarly be provided between Peer Groups.

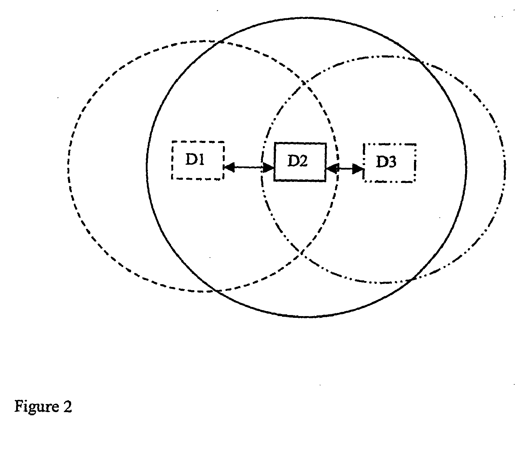

Where component devices are within communication range of one another, in-range communication links provided between such component devices enable the component devices to directly communicate with one another. This is shown in Fig. 2, where component device pairs D1-D2 and D2-D3 can communicate directly to each other as a result of being within communication range of one another, respectively, wherein the communication range encompassing D1 is shown in the dashed line circle and the communication range encompassing D3 is shown in the dashed-dotted line. The solid line circle in Fig. 2 represents the communication range of device D2. Referring still to Fig. 2, it is apparent that D1 and D3 cannot communicate directly to each other, as each is out of the other's communication range. Direct, in-range communication between component devices use asymmetric and symmetric cryptographic techniques referenced above, which can be provided by protocols such as Pretty Good Privacy (PGP) and Anonymous but Accountable Self-Organizing Communities (AASOC) protocols. Such techniques and protocols help to establish trust between component devices, and help to securely transmit arbitrary data between the component devices that are within communication range of one another.

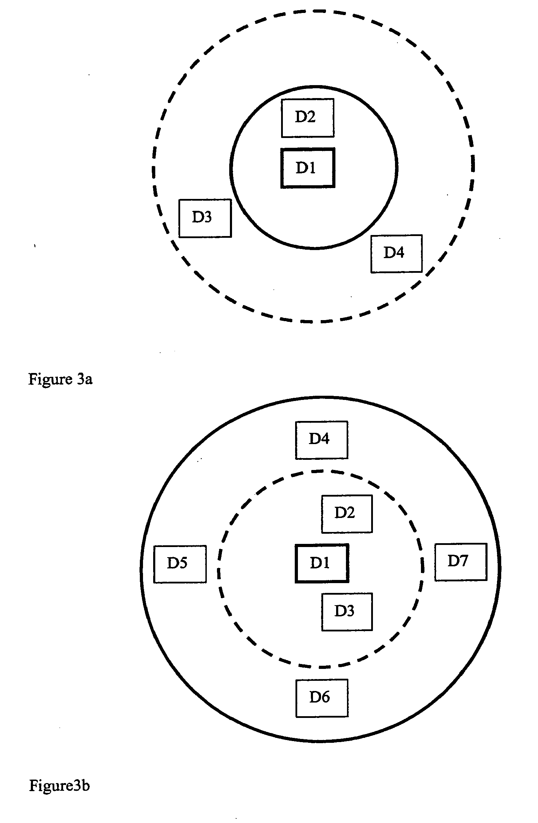

The communication range of a component device can be predetermined, or can be dynamically adapted, according to the requirements of the overall medical device. For example, if a component device can communicate directly with only a few other devices (for example, less then a predetermined number of devices), then its maximal communication range may be increased. On the other hand, if a device can communicate directly with many other component devices (for example, more than a predetermined number of devices) then it's communication range may be decreased, to save energy by communicating to shorter distances. Fig. 3a shows an example of when the component device D1 should increase its communication range from its original range (solid line) to a new larger communication range (dashed line). Such an increase in communication range is often appropriate when the required number of in-range devices is at least two, but communication with more than two devices increases the effectiveness, or sensitivity, of the network overall. Fig. 3b shows an example of when the component device D1 should decrease its communication range from its original communication range (solid line) to a new smaller communication range (dashed line). Such a decrease in communication range may be appropriate if the number of in-range devices is as much as five devices, for example, but the effectiveness, or sensitivity, of the network would be enhanced by limiting communication to fewer devices. The actual communication range may also depend on the physical parameters of the communicating component devices and/or the parameters particular to the communication, such as the communication medium (radio frequency, infrared, wired, etc.), the communication hardware (antenna, wire, IR sensor type, etc.), the available energy for communication (less available energy generally means smaller communication range preferred), the maximal transmission error bit rate allowed, etc., or other parameters of the various component devices involved. The component devices can change their communication range, for example, by communicating with less or more power, by changing the form of communication they use, by changing their physical location, or by changing other parameters discussed herein.

Referring again to Fig. 2, where the pair of component devices D1 and D3 are out of communication range of one another, then out-of-range, or multi-hop, communication between such component devices occurs to link the otherwise out-of-range component devices D1 and D3 with one another. The out-of-range communication between component devices D1 and D3, for example, occurs via one or more intermediate component devices, in this case D2, that link the otherwise out-of-range component devices D1 and D3 with one another. Out-of-range communication between component devices may be organized and maintained by pipeline protocols such as SPP within the network.

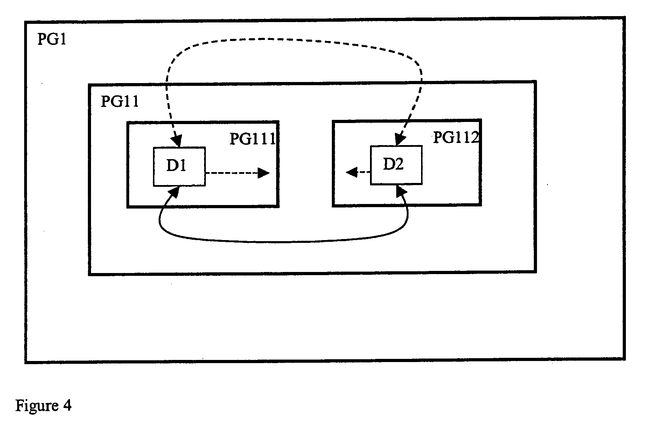

Generally, component devices can communicate with each other if they are in the same level peer group, i.e., PG11 or PG12. But all component devices within a network are in the main overall Peer Group (PG1 in Fig. 1.). Therefore, any component device can ideally communicate with any other component device directly or indirectly in the overall Peer Group. Localizing communications between component devices is preferable however, to optimize the efficiency of the network. To localize the communication of the component devices, the devices should ideally communicate with devices that are within the lowest common level of peer group in the hierarchy of the network. Figure 4 thus shows that component devices D1 and D2 communicate with one another via peer group PG11 rather than through the overall Peer Group PG1. Because the peer group PG11 is the lowest common level of peer group with which both component devices D1 and D2 share, the component devices D1 and D2 preferably do not communicate with each other through sub-peer groups PG111 or PG112, even though such sub-peer groups comprise a part of the overall Peer Group PG1 as well. In any event, component devices D1 and D2 should therefore communicate through PG11 and not through PG1, to make the communication as localized and efficient as possible.

Asymmetric cryptography generally establishes trust between component devices within a respective peer group, whereas symmetric cryptography generally authorizes data exchange between component devices within the respective peer group after trust has been established between component devices using asymmetric cryptography initially. Survivable Pipeline Protocols (SPP), on the other hand, are used to execute and maintain prioritized tasks, or functions, and communication pipelines appropriately within the network. SPP are also used to update the trust relationship between component devices by establishing the hierarchy of self-organized component devices (i.e., by creating the hierarchy of various levels of peer groups and maintaining local information about other component devices). Once the component devices within a respective peer group level have performed their respective tasks or functions, then the task of the respective peer group is executed. Security and anonymity of the communications are enhanced by the cryptographic, PGP, AASOC based communication methods employed within the network of the medical device according to the systems and methods of the invention.

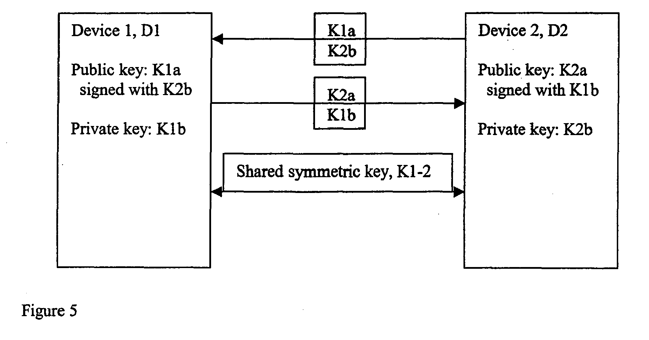

According to PGP cryptography, as shown in Fig. 5 for example, component device D1 is provided with a public key K1a and a private key K1b, and device D2 is provided with a public key K2a and a private key K2b. When the public key K1a of the component device D1 is signed with the private key K2b of the component device D2, as evidenced by the arrow having K1a atop K2b from component device D2 to D1, trust is established from the component device D2 to D1, for example. Similarly, when the public key K2a of the component device D2 is signed with the private key K1b of the component device D1, as evidenced by the arrow having K2a atop K1b from component device D1 to D2, trust is established from the component device D1 to D2. Once such trust is established between component devices, two-way communications between component devices D1 and D2 is readily authorized. Other component devices have similar public keys and private keys associated therewith for establishing trust and communication relationships therebetween. A network of trusted component devices of the medical device is thus similarly established by having component devices sign the public keys of trusted component devices with their own private keys.

Once the public key-private key relationship between devices is established, a symmetrical key may be generated to communicate between component devices, such as symmetrical key K1-2 generated between component devices D1 and D2 as shown in Fig. 5. The symmetrical key, when generated, enables faster, simpler and more secure communications between trusted devices, such that a component device can communicate with any other trusted component device within the overall Peer Group PG1. Encoding and decoding messages with a symmetrical key is typically simpler and more efficiently processed between component devices than are messages using the public key/private key algorithms, cryptography or protocols therein, thereby aiding the efficiency of communications between component devices once a symmetrical key arrangement has been established.

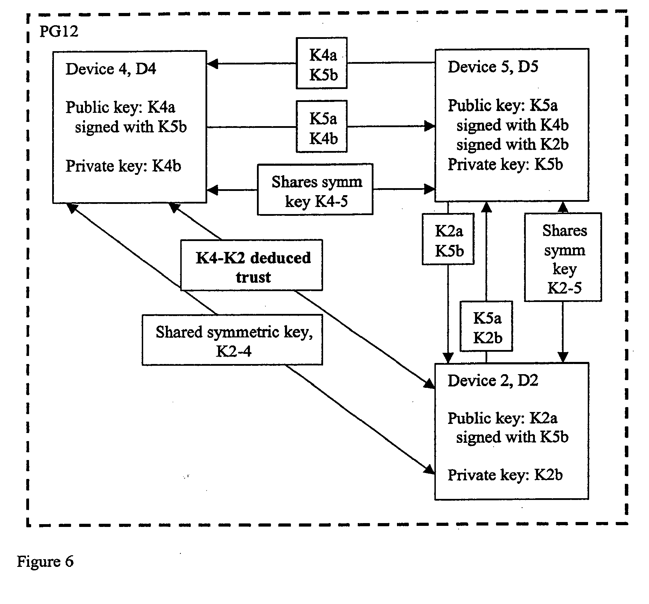

Referring now to Fig. 6, for example, Peer Group PG12 is shown in dashed lines, component devices D4 and D5 are indicated as having a symmetric key K4-5, showing the trust established and enabling communication therebetween. Similarly component devices D5 and D2 are indicated as having a symmetric key K5-2 showing the trust established and enabling communication therebetween. On the other hand, component devices D4 and D2 are shown as using the public key / private key relationship (K4a & b, and K2a & b) to enable communication therebetween. In this instance, however, public key K2a of device D2 is not signed by the private key K4b of device D4, and the public key K4a is not signed by the private key K2b. Trust between component devices D2-D4 can nevertheless be deduced, as shown by the arrow K4-K2 because the component device D4 trusts in D5, the component device D2 trusts in D5, and D5 trusts both D4 and D2. Therefore D4 and D2 conclude, based on the respective signatures of the public keys K4a and K2a by private key K5b, that component devices D2 and D4 can trust each other. A symmetric key K2-4 is thus generated between component devices D4 and D2 once sufficient trust has been established therebetween using the public key / private key relationship as before described. This indirect ("deduced") trust establishment enables some component devices to refrain from signing and storing the public keys of all of the other component devices while still participating in the establishment of trust between certain devices, whereas only those component devices between which communications frequently occur tend to sign and store the public keys of the other component devices.

Using PGP cryptography and AASOC with the various component devices comprising various levels of the peer groups of the network helps further assure the internal and external accountability of the data communicated between the component devices. Using PGP and AASOC further helps maintain and secure the privacy and confidential nature of the data communicated therebetween the component devices and levels of peer groups of the network. Sensor networks, especially in medical applications, gather much personal and highly sensitive information from the patient and the environment, which emphasizes the desirableness of protecting the information stored in the network, and of securing the anonymity of the communications processed by the network. PGP and AASOC also preferably provided increased accountability against malicious users and devices.

Because the component devices within the network are relatively simple, having relatively small computational capacities (as compared to PC's, PDA's or mobile phones, for example), with relatively limited power supplies, any algorithms applied by the devices to provide security within the network are ideally selected and implemented to minimize energy consumption and computational resource requirements. PGP cryptography tends to achieve these goals using the asymmetric and symmetric key cryptography approach detailed above, wherein asymmetric public key-private key relationships initiate the trust relationship between devices, which is then replaced with the generally faster, more efficient symmetrical keys and algorithms associated therewith. The private key signature of component devices on the public keys of other component devices helps to ensure the identity and security of such devices and the information or data communicated among the devices, which increases the reliability and efficiency of the network overall. Risk of failure of the network and medical device therefore, as where trust between devices is disrupted, can be minimized even further by implementing more expensive and computationally elaborate algorithms tailored to the capabilities of the component devices if desired.

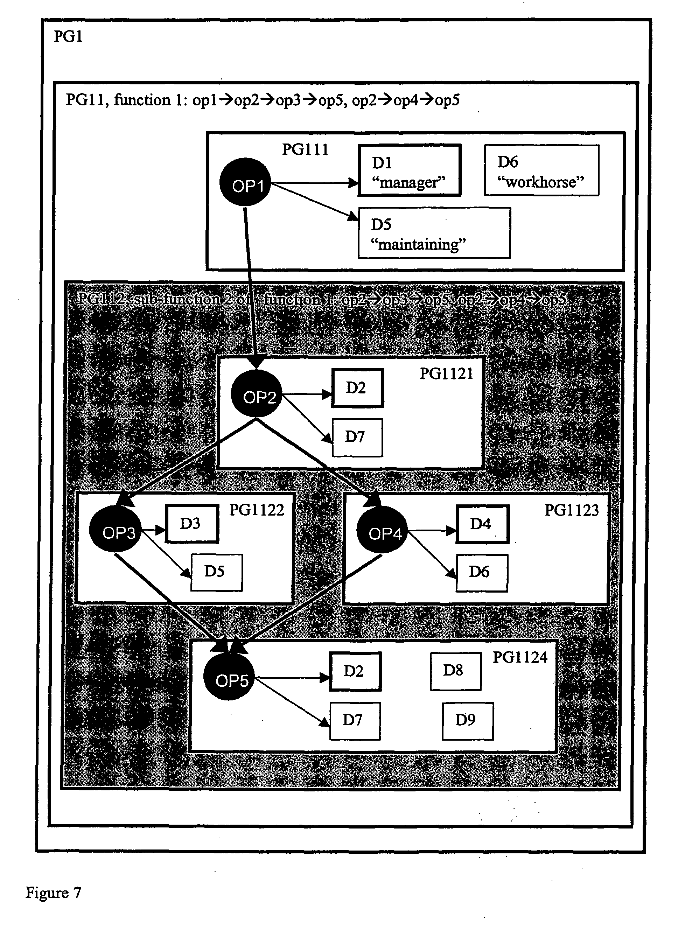

Referring now to Fig. 7, the execution of a task or function of a medical device and the various created levels of peer groups contributing to the execution of that task or function are shown. The overall Peer Group is PG1. Function 1 is shown as assigned to peer group PG11. Sub-function 2 of function 1 is shown as assigned to sub-peer group PG112. Peer group PG11 is further comprised of sub-peer groups PG111 and PG112, and sub-peer group PG112 is still further comprised of sub-sub-peer groups PG1121, PG1122, PG1123 and PG1124. Function 1 contains five operations, shown as circles OP1 to OP5. After the first operation OP1 is executed (for example, the sensing of some physiological parameter) the result is sent to PG1121 to be incorporated into the second operation OP2. After OP2 is executed the result is sent to PG1122 to be incorporated into OP3 and to PG1123 to be incorporated into OP4 simultaneously. After the execution of OP3 and OP4 the results are sent to PG1124 to be incorporated into OP5. In the course of the execution of OP1-OP5 a therapeutic effect to the patient may be delivered. The therapeutic effect may be some amount of drug, action or stimulus delivered to some location in the patient's body; where the amount delivered and the place of delivery may depend on the outcome of the previous operations of the function. Thereafter, feedback indicating that the assigned function has been executed is sent to the device D1, i.e., the "manager" device responsible for co-ordinating and scheduling all of the component devices D1-D3 to perform the respective tasks or functions to execute completely the first operation of function 1. (The concept of "manager" devices is discussed in greater detail further below). Referring still to Fig. 7, bold arrows show data propagation between component devices and the various levels of the peer groups comprising the medical device, wherein the overall Peer Group PG1 is represented as the largest rectangle, the peer PG11 is represented as the second largest rectangle, the sub-peer group PG111 is represented as the smaller interior rectangle all of which correspond to operation OP1. The sub-peer group PG112 is represented as the darkened interior rectangle. Sub-peer group PG112 is further comprised of a series of sub-sub-peer groups PG1121, PG1122, PG1123 and PG1124 that are represented as smaller rectangles within the darkened sub-peer group PG112 rectangle. The component devices D1-D9 are represented as the smallest rectangles in Figure 7. Of the various levels of peer groups depicted in Figure 7, PG1 and PG11 are responsible for the organization of the eventual execution of function 1 upon completion of sub-function 2, while PG111, PG112 and PG1121-PG1124 are responsible for the execution of specific operations OP1-OP5 and sub-function 2 that comprise part of the overall function 1 of the medical device.

Referring still to Fig. 7, the component devices comprising a peer group can fulfill any of three different roles. For example, component devices are either "workhorse" devices, "maintaining" devices, "manager" devices, or some combination thereof. "Workhorse" component devices are responsible for executing the assigned task or function of the respective peer group once data to be processed thereby is received. Every component device in a peer group may act as a "workhorse" device. "Maintaining" devices receive data from a previous operation and distribute the data to one or more subsequent "workhorse" devices in the peer group for processing that data; and maintaining the roles within the peer group. "Manager" devices are "maintaining" devices that also schedule data allocation and processing within the peer group, recruit new devices to assist in the execution of assigned tasks for the peer group if needed, and organize the assignment of successor tasks (or operation) to other "manager" devices in various levels of component devices within the peer group. Among other things, the "manager" devices help simplify the transmission of data from one level to another in those cases when input data from multiple predecessor operations or sub-tasks is received, as in operation OP5 in Fig. 7, for example. In such cases the corresponding data packages need to be sent for processing to the same "workhorse" device within the peer group, where the packages are typically consolidated before processing commences.

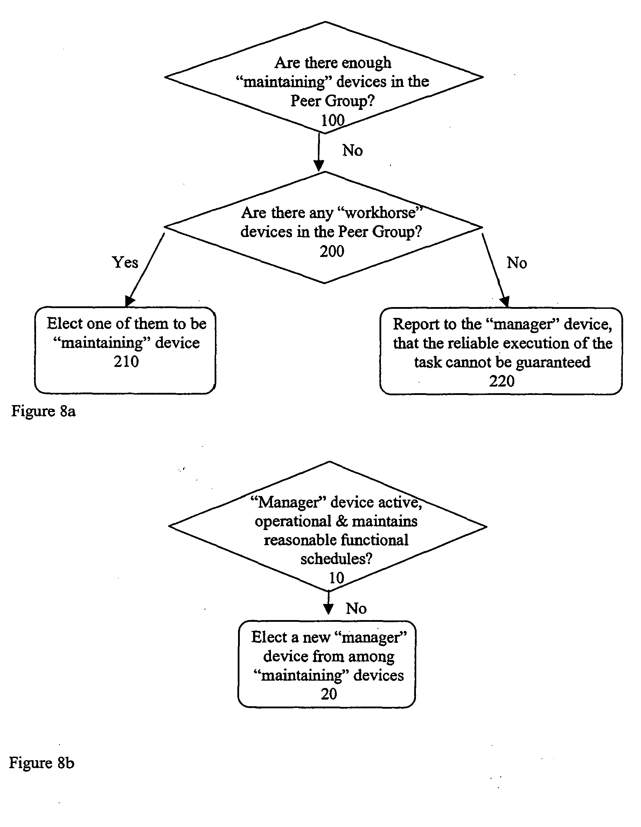

Figure 8a shows a process of ensuring that there are a sufficient number of "maintaining" devices within the peer group to receive and distribute data effectively to "workhorse" devices for timely processing of the data. If there are not enough "maintaining" devices in the peer group to safely meet the task's reliability requirements within the peer group, then "workhorse" devices are recruited from within the peer group to become "maintaining" devices. If "workhorse" devices are not available to recruit from within the peer group, then a notification is sent to the "manager" device of the respective peer group that the execution of that peer group's assigned task, function or operation cannot be guaranteed with the current devices available in the peer group. Figure 8b shows a process of recruiting additional "manager" devices from among the available "maintaining" devices when a prior "managing" device fails to respond or function appropriately.

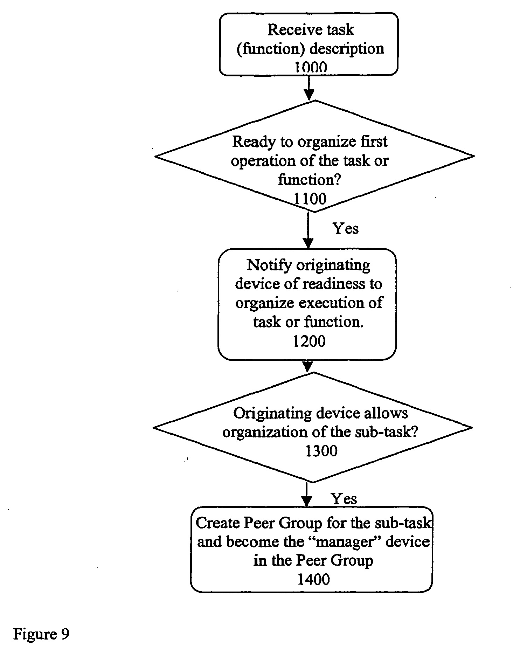

Fig. 9 is a flowchart illustrating how the organization of a task or function starts, wherein only the term task is used hereafter although functions and the various levels of tasks, operations or functions are understood to be encompassed in the description set forth herein. The tasks to be organized and executed by the network of component devices are advertised within the network. In step 1000, a description of the advertised task is received by the various component devices in the network. The description of the advertised task includes a description of the operations required to perform the task, the preferred data paths between the operations, reliability requirements, and required execution times (for example, latency and restart times). In step 1100, a component device receives such an advertisement, and determines whether it will accept the advertised task. If the device decides to accept the advertisement, then in step 1200 the accepting device sends a notification of its intent to accept and organize the execution of the advertised task to the component device from which the advertisement originated. Several of the component devices may be capable of performing the advertised task, thus several notifications of intent to accept may be forthcoming from the various capable component devices. However, because a task is preferably assigned to one peer group, preferably only one component device is ultimately authorized to organize the advertised task, the component device that originally advertised the task decides which notification of intent to accept the task should be accepted, and thus which component device is ultimately authorized to organize the advertised task. This determination is made based upon the specifics of the tasks and the past performance on a similar task of each component device volunteering for the tasks. In step 1300 the originating device determines which device is authorized to accept and organize the advertised task. Once a component device receives a message from the advertising device authorizing the starting and organizing of the advertised task, then in step 1400 that authorized device must start to organize the advertised task for execution thereof (or sub-task). The first step of organizing a task for execution thereof is the creation of an appropriate level peer group that will be responsible for the eventual execution of the advertised task (or sub-task).

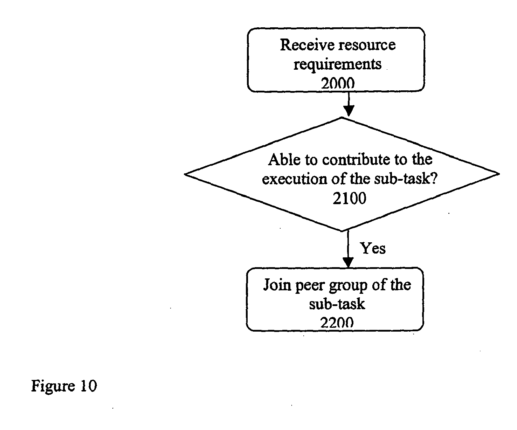

If a peer group is created to execute a task directly, then the resource requirements of the execution of that task are advertised. The operations performed by the created peer group level may be a single operation or may be some consecutive operations. In any event, the advertisement of resource requirements by the created peer group contains the same or similar information as that provided in the task description detailed above in order to attract multiple devices to respond to the advertisement and join the peer group. Fig. 10 provides a flowchart illustrating the case where a component device determines whether it is capable of performing the advertised task, taking into account resource requirements; and whether it should join the created level peer group authorized to perform the task associated therewith. As shown in Fig. 10, the advertisement setting forth the description of the resource requirements for performing a task are received by a component device in step 2000. Then, in step 2100, the component device determines whether it can contribute to the execution of a task within the targeted reliability and frequency parameters set forth by the advertised resource requirements. If so, then in step 2200 the component device joins the created peer group authorized to perform that task. If not, then further recruitment of component devices is needed in light of the advertised resource requirements. Once created, a peer group needs a sufficient amount of component devices to execute all of the operations required to complete the overall task within targeted reliability and frequency parameters. Where additional data to be processed by a component device is generated before preceding data has been fully processed by the same component device, then a backlog of data and processing could occur with undesirable reliability and frequency effects. In this case, pipeline operations may be used between component devices to continue to process data subsequently generated even as a prior task, function or operation processing is underway. The use of such pipeline operation means that the devices within a common level peer group that was created and authorized to perform a task may start processing subsequent data even while the previous data is not fully processed. Fewer component devices are required to process increased volumes of data as a result of the pipeline operations.

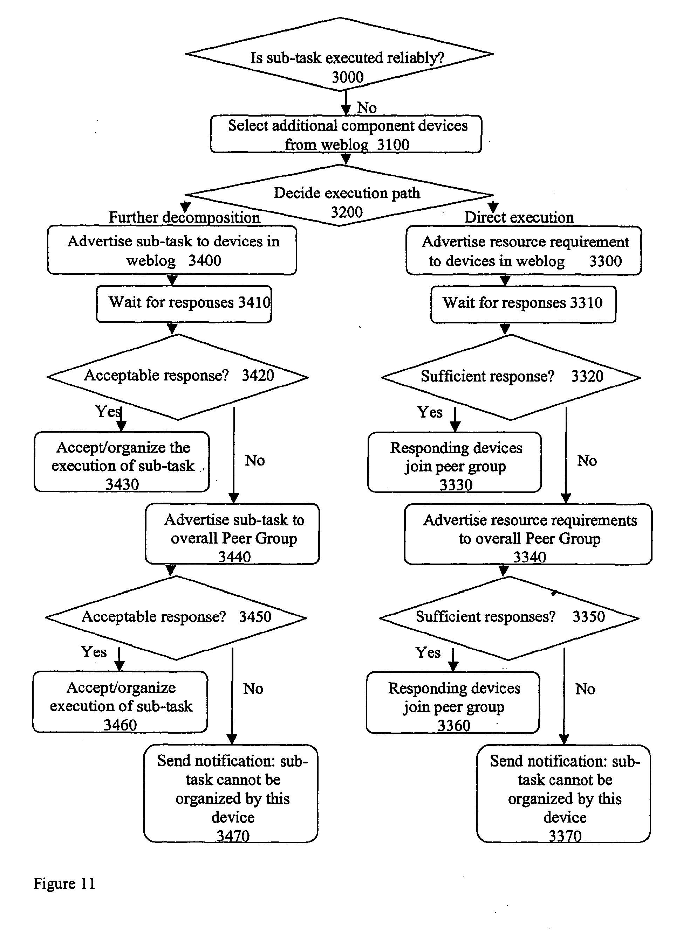

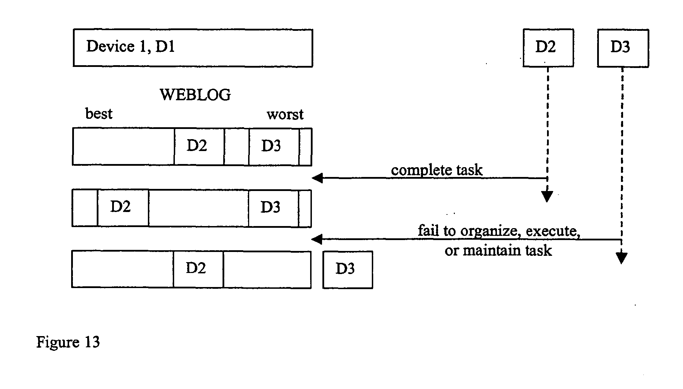

Fig. 11 provides a flowchart illustrating the management of a task delegated to a peer group. The "manager" device of the peer group executes the steps provided in the task management flowchart of Fig. 11 regularly. In step 3000, the "manager" device determines whether a task is being executed reliably, or if the timing constraints within which the task is to be performed are not being satisfied by the current component devices existing within the peer group. If the task is not reliably or timely executed, then in step 3100 the "manager" device selects additional component devices from a weblog associated therewith. The weblog, described in more detail further below with respect to Fig. 13, is a list of component devices that are ranked or ordered based on their reliability to perform a given task. The additional component devices are preferably not in the same peer group and their recruitment thus increases the overall resources and capability of the peer group.

After selecting the additional reliable component devices, then in step 3200 the "manager" device decides whether the task of this peer group should be executed directly via steps 3300-3370 by newly recruited component devices in collaboration with existing devices within the existing peer group, or whether the task of this peer group should be broken up or decomposed into smaller sub-tasks via steps 3400-3470. If the task should be executed directly by the existing peer group, then in step 3300 the resource requirements of the task execution are advertised to the various devices. If the task of the peer group should be decomposed, then in step 3400 the task of the peer group is advertised to the selected devices within the peer group.

When direct execution is pursued, then in step 3310 the "manager" device waits for responses to the advertisement from the available devices. If the manager device receives satisfactory responses from various component devices within a given time interval in step 3320, then in step 3330 the various responding component devices join the peer group to assist in executing the intended task of the peer group. On the other hand, if unsatisfactory responses were obtained in step 3320, then, in step 3340, the resource requirements of the intended task to be executed are advertised to all of the devices in the overall Peer Group.

If satisfactory responses to the resource requirement advertisement of step 3340 are received in step 3350, then those responding component devices are joined with the other devices in the peer group in step 3360 to assist in executing the intended peer group task. Otherwise, if unsatisfactory responses continue to be obtained in step 3350, then a notification is sent by the "manager" device in step 3370 that the task cannot be organized or executed. The notification is sent to the component device from which the advertisement of this task was originally received. When further decomposition of a task is pursued prior to execution of the task, then in step 3410 the "manager" device waits for responses to the advertisement from the available component devices selected from its weblog.

If the "manager" device receives satisfactory responses from various component devices within a given time interval in step 3420, then in step 3430 the various responding component devices are accepted and join the peer group to assist in executing the intended task of the peer group. On the other hand, if unsatisfactory responses were obtained in step 3420, then, in step 3440, the intended task to be executed is advertised to all of the devices in the overall Peer Group. If satisfactory responses to the task advertisement of step 3440 are received in step 3450, then those responding component devices are accepted and joined with the other devices in the peer group in step 3460 to assist in executing the intended peer group task. Otherwise, if unsatisfactory responses continue to be obtained in step 3450, then a notification is sent by the "manager" device in step 3470 that the task cannot be organized or executed. As before, the notification is sent to the component device from which the advertisement of this task originated.

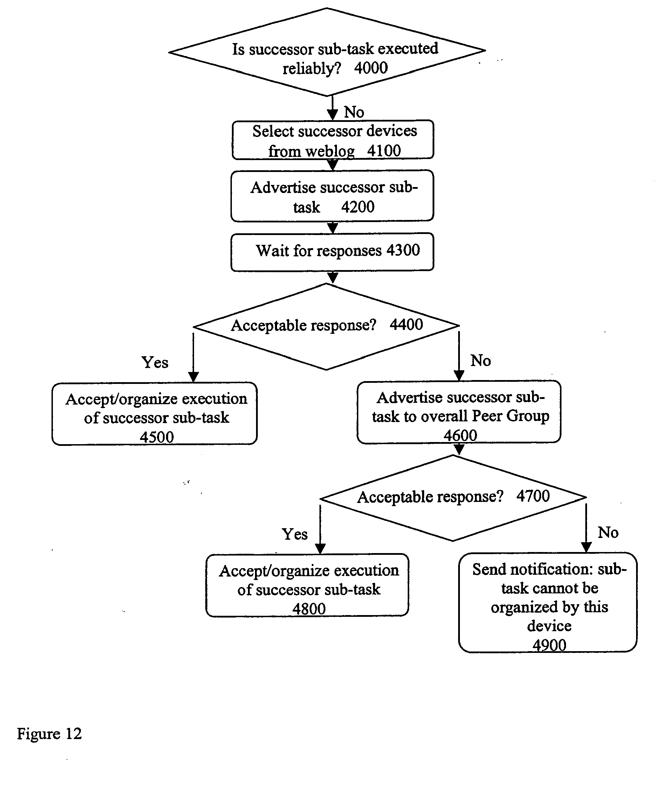

Fig. 12 shows a flowchart illustrating the maintenance and organizing of a successor task. A successor task is understood to be the next task to be executed after completion of all required preceding tasks. According to Fig. 11, a peer group is created and responsible for the execution of a particular task. Typically such tasks comprise underlying levels of some bigger task to be executed by a subsequent or higher-level peer group, or the overall Peer Group, of the medical device. Therefore successful execution of the tasks of underlying peer groups increases the likelihood that execution of the bigger task will not fail after the underlying peer group tasks have been executed. Upon completion of the underlying task, the underlying peer group is further responsible for finding a component device that will initiate the organization and maintenance of the first part of the successor task. In other words the underlying peer group is not responsible for organizing the entire successor task. Rather, the only responsibility (in addition to executing the task for which it was created) is to recruit and communicate with another device that will organize the reliable execution of some first part of the successor task. This makes the network of devices and task allocation modular, distributed and robust. Task execution in the network of devices thus never depends on only one device.

As shown in step 4000 of the flowchart of Fig. 12, the "manager" device regularly verifies that the execution of some first part of the successor task is reliably and timely executed. If the verification fails, or a notification is received that the successor task cannot be organized or executed reliably, then in step 4100 the "manager" device selects some successor devices from its weblog and in step 4200 advertises the successor task to those component devices identified in the weblog. In step 4300 the "manager" device waits for a response from the successor devices regarding the advertisement. If the "manager" device receives an acceptable response in step 4400, then in step 4500 a successor device is accepted and execution of the successor task is organized and execution thereof begun. If, on the other hand, the "manager" device does not receive a satisfactory response to the advertisement in step 4400, then in step 4600 the advertisement is sent to all devices in the overall Peer Group. If the "manager" device receives a satisfactory response to the advertisement in step 4700, then in step 4800 the successor device is accepted and organization and execution of the first part of the successor task is begun. If, however, the "manager" device still does not receive a satisfactory response to the advertisement in step 4700, then in step 4900 the "manager" device sends a notification to the originating device from which the "manager" device received the task description that the organization and execution of some first part of the successor task cannot be reliably arranged by the "manager" device. The originating device that receives the notification should recruit another device to arrange the organization and execution of the successor task.

Referring now back to Fig. 7, as one illustrative example of organizing the function of the medical device, D1 received the description of function 1 and was thus recruited to start to organize that function. D1 created peer group PG11 and decided that OP1 could not be directly executed by itself. Therefore D1 created PG111, selected some partner devices from its weblog, and sent the resource requirements of OP1 to those devices. Furthermore D1 decided that the function 1 needed to be further decomposed, and therefore advertised the remaining tasks (OP2-OP5) with the fact that it has an assigned peer group PG11. If needed then it sent the advertisement to all other devices in the overall Peer Group PG1. Finally D5 and D6 joined the peer group PG111 and D1 became the "manager" device of the peer group PG111. During this time, D2 responded to D1 regarding D1's task advertisement with the intent to organize the whole successor task. D1 accepted D2's intent, therefore D2 created the peer group PG112. D2 decided that OP2 cannot be executed directly by itself, therefore it created PG1121, selected some partner devices and advertised the resource requirements of OP2. D2 also decided that the other parts of the task OP2 should be further decomposed, therefore D2 advertised the remaining task with the fact that it has an assigned peer group PG112. Similarly D3, D4, and D5 organized the execution of OP3, OP4, and OP5 and the other devices joined the created peer groups. Finally the execution of the whole function is reliably and timely executed and maintained by the devices with the SPP protocol.

If a device breaks down or somehow disconnects from the network, then the other devices that relied on its resources in the execution of a task will notice it. If a device can detect that it will likely break down, then it should send a notification to other devices that it will break down, for example, or that its energy source is depleting. When a device does not answer to messages then it is considered to be not operational and other devices will not count on that device until the device starts to answer messages.

If a new device is introduced into the network, then that device can join any peer group that needs resources to reliably execute, or start to organize an advertised task. In order to ensure the required security of the system and the prevention of malicious intrusion, the new device may need to provide proof of its right to join the network of devices. Also, for example in an AASOC system, procedures typically exist to disqualify devices that do not operate according to the rules of the overall medical device. This further increases the security of the medical device even against malicious devices.

Fig. 13 illustrates schematically a self-organized ranking of the communication and task execution reliability pattern of component devices D1-D3. The reliability patterns are illustrated as maintained in a weblog (W) of each component device according to the systems and methods of the invention. As shown in Fig. 13, a weblog (W) of trusted component devices is maintained by each device based on the public key-private key, or symmetrical key, relationship established between devices as detailed above. Each device listed in the weblog W of another device thus has some degree of reliability for communication and task execution with the corresponding component device. However, the weblog W of each device prioritizes the list of trusted devices such that those most-trusted devices are listed first, and least trusted devices are listed last within the weblog W. Lesser-trusted devices are thus listed in successive order between first and last listed devices on the weblog list of a respective device.

In practice, referring still to Fig. 13 a weblog W for a component device D1 is shown. After a peer device D2 has completed a task that it received from device D1, the reliability of D2 according to D1 may increase. That reliability is represented in the weblog W of device D1 by placing device D2 in a first position in the weblog W of D1. Meanwhile, were device D3 to fail to execute a task received from device D1, then reliability of D3 according to D1 may decrease. As a result, device D3 is placed below device D2 on the weblog W of D1. If D3 continues to fail to execute a task communicated to it from D1, then D3 will eventually be removed from the weblog W of device D1, such that, in this instance, D1 will not send task advertisements directly to D3. By listing the various trusted devices by the degree of trust or reliability of a device, the efficiency of the processing and communicating data through the network of the medical device can be enhanced. Also, note that if the resources of a device (e.g., memory) enable the storage and update of more weblogs then that device may have separate weblogs for each type of task or communication and can select a device from the appropriate weblog when needed.

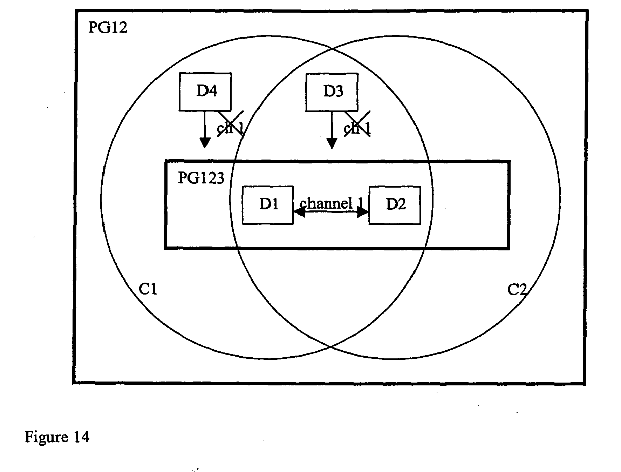

Fig. 14 illustrates an embodiment of a local communication scheme between component devices within a Peer Group 12 according to the systems and methods of the invention. The communication scheme set forth in Fig. 14 illustrates peer devices D1-D4. Devices D1 and D2 prefer to communicate with each other directly as they are within each other's communication range (C1 and C2 respectively). Note, that a communication range (as it is a physical notion) is not limited by the logically existing peer groups. Fig. 14 illustrates this concept as D1 and D2 are within the peer group PG123 of Peer Group PG12, whereas D3 and D4 are not within the peer group PG123 but are within the overall Peer Group PG12. Nevertheless, D3 and D4 are each within one of the communication ranges C1 and C2 that also include D1 and D2. To avoid communication interference between devices, communications between devices are preferably scheduled such that, when two devices within each other's communication range (C1 and C2) communicate directly using a specific channel, for example channel 1, then other devices within the communication range of those devices (C1 and C2) do not use the same channel.

For example, referring still to Fig. 14, devices D1 and D2 communicate directly to one another via channel 1 within the communication ranges C1 and C2. Thus, device D3 cannot communicate to any devices using channel 1 during the communication of D1 with D2 because D3 is in the communication ranges C1 and C2 as well. Because D3 is within the communication range of both D1 and D2 each of D1 and D2 can schedule communications directly with D3 so that communication interference can be avoided easily. On the other hand D4 is out of the communication range of D2, therefore D2 is typically not even aware that D4 can interfere with D1, but D2 and D4 cannot communicate at the same time to D1 using the same channel. Furthermore, if D1 communicates on channel 1, then D4 cannot communicate to any device using channel 1 during that time because it would interfere with D1's communication. Therefore D4 must schedule its communication not to use channel 1 when, for example, D2 communicates with D1 on channel 1. If communication is scheduled and allocated in a similar way as the tasks are allocated and scheduled with respect to the various levels of component devices using SPP, for example, then communications interference can be minimized. Using such a communication scheme, as illustrated in Figs. 7 and 14 for example, permits more reliable communications to occur between devices while minimizing interference therewith. As a result, the tasks or functions to be undertaken by the component devices within the respective peer groups may be more reliably performed.

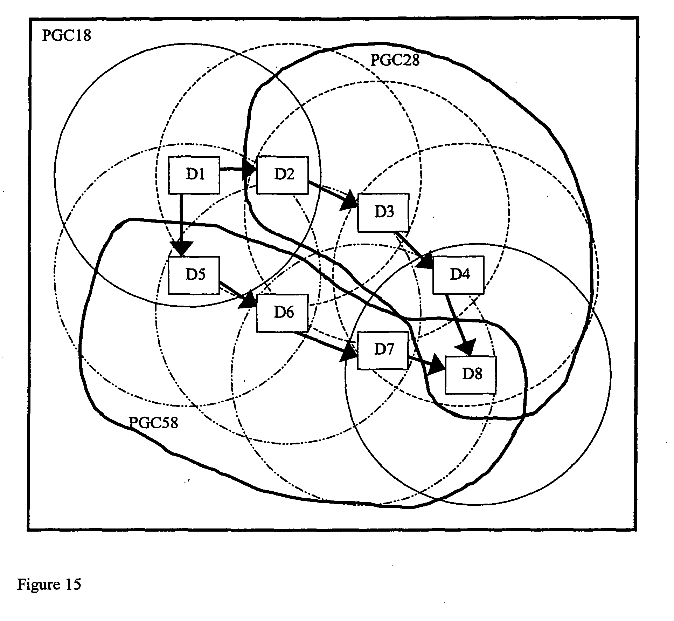

Fig. 15 illustrates one embodiment of a routing scheme for communicating data from component device D1 to component device D8 according to the systems and methods of the invention. In Fig. 15, devices D1-D8 have communication ranges represented by circles. D1 and D8 are out of each other's communication range, represented by symmetric circles with solid lines. The communication ranges of devices D2-D4 are illustrated by dashed circles, while the communication ranges of devices D6-D7 are represented by dashed-dotted circles. There are three peer groups PGC18, PGC28 and PGC58 in Fig. 15, wherein each peer group is created for communication tasks. The large rectangle represents the peer group PGC18, which is created for the communication between devices D1 and D8. The two hand-drawn bold lines represent peer groups PGC28 and PGC58, respectively created for communication between D2 and D8, and D5 and D8.

Referring still to Fig. 15, D1 wants to communicate with D8, but D1 and D8 are out of each other's communication range. Therefore D1 creates the peer group PGC 18 to localize communications between D1 and D8, whereby only those devices that join this peer group can contribute to the communication. D1 does not know any route to D8 and therefore advertises this communication task to all devices. Devices D2 and D5 answer the advertisement as they know routes to D8. D1 accepts these responses. D2 and D5 therefore create the peer groups PGC28 and PGC58 to localize their communications with D8, and send the communication resource requirement advertisement to the devices in the route to D8. That is D2 sends the advertisement to D3 and D4, while D5 sends the advertisement to D6 and D7. Finally D4 and D7 are within the communication range (symmetric circle with solid lines) with D8. D8 is thus notified through D4 and D7 that D1 wants to communicate with D8. Devices D1 and D8 now can establish trust between each other with their public-private key pairs as described hereinabove. In practice, therefore, the medical device comprised of a network of various levels of peer groups (PG1, PG11,..,PGC18,PGC28,..) comprised of different component devices (D1-D8) according to the systems and methods of the invention, would thus communicate data regarding sensed physiological parameters, compute, store or distribute such data, and then initiate a response to such data to address or remedy a condition in a patient based on the communications exchanged within the network.

With respect to the Survivable Pipeline Protocols discussed above, collaborative device networks create challenging resource allocation and scheduling problems. For example, the challenge posed by the interference of the communication of component devices, discussed above with respect to Fig. 14, can be solved, for example, by distributing sensory information through designated low bandwidth RF channels between component devices or various levels of component devices in the network. Distributed pre-processing of the information or data obtained at each stage can lower the amount of information that is being communicated between component devices or the various levels thereof to render the communication between component devices and levels of the network even more efficient, if desired. In this case, the pre-processing tasks of possibly heterogeneous component devices, each of which may have limited computational capacity, may have to be completed to render the network operational. Further, multi-hop communication channels between distant component devices are preferably maintained in order to enhance the efficiency of the network. The Survivable Pipeline Protocol (SPP) used in the context of the systems and methods of the invention, provides the framework of algorithms within the component devices to perform multi-hop message routing, local communication, task allocation and scheduling for a medical device comprised of a network of a multitude of component devices according to the systems and methods of the invention.

SPP has multiple benefits, which render it accommodating for use with the networked medical device of the instant invention. For example, SPP:
- recruits component devices in a population of heterogeneous and low processing capacity units to accomplish complex and energy-expensive tasks (e.g. encrypting/decrypting messages for increased security) that otherwise are beyond the capabilities of individual units
- allows component devices with communication capability (e.g. RF communication) to reduce interference by effectively scheduling communications
- reduces communication traffic by distributed preprocessing of the data to be communicated
- supports multi-hop communication channels between distant component devices
- adapts to dynamic changes in the device population, brought about by the loss or introduction of units or by changes in the performance of individual units

The systems and methods of the invention described hereinabove thus provide a means for sensing physiological parameters in, on or around a patient and achieving a therapeutic effect for the patient with a medical device that has functional robustness due to its construction as a network of relatively simple component devices that are able to self-organize into a dynamic, collaborative hierarchy to accomplish various levels of tasks or functions. The failure of any one component device therefore does not significantly impact the performance of the network, but rather reduces the functional capability of the entire network of the medical device by a small amount only. Ideally, therefore, the medical device will not experience complete loss of function even when one or some of the component devices comprising the network fail. Each component device can have a relatively inexpensive and simple structure that individually performs simple functions but that collectively, when assembled within the network, is able to contribute to the performance of more complex functions. The small size of the component devices reduces volumetric intrusion of a patient if implanted or attached to the body of the patient. The small size of the component devices also accommodates dispersion of the devices throughout the body, or throughout the environment in which the body is located, to sense or achieve a therapeutic effect in multiple locations simultaneously and in concert.

A neural prosthesis may be constructed based on the principles described in the preceding paragraphs. Such a neural prosthesis performs a vital function by replacing, reinforcing or repairing a neural function. The replacement, reinforcement or repair of neural function is achieved by therapeutic component devices that have the ability to stimulate or depress the function of certain components of the nervous system. To provide such stimulation or other functions to the patient, these therapeutic component devices receive information from multiple sensory and computational subunits of the same neural prosthesis. In other words, the operation of the therapeutic devices is embedded in this sensory and computational network of devices. This sensory and computational network of devices helps repair the coupling between the patient and its environment that was otherwise compromised by neural damage.

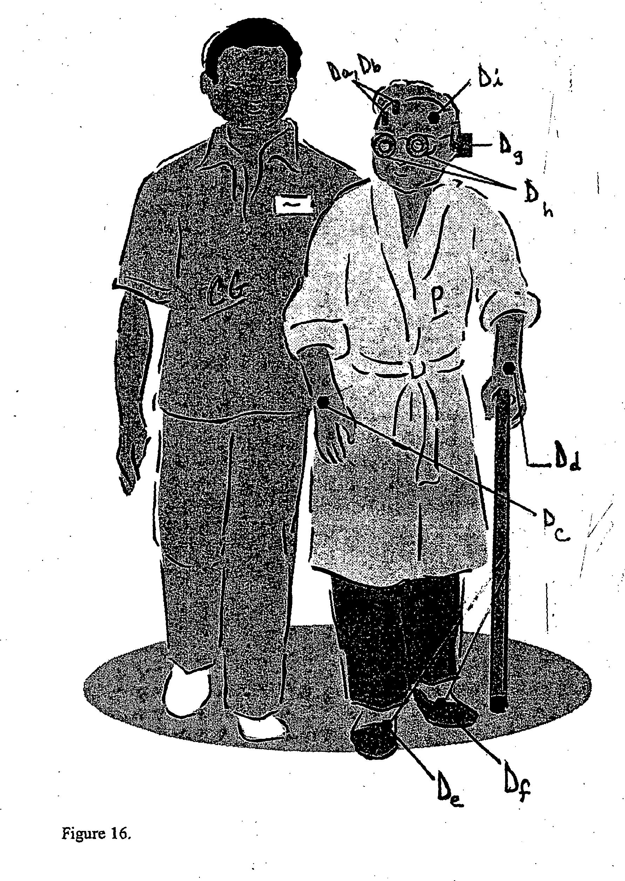

Figure 16 illustrates one embodiment of a network of component devices for sensing various parameters and delivering a therapeutic effect based thereon according to the systems and methods of the invention. This figure shows a movement disorder patient (P) (e.g. a Parkinson's Disease patient) using an embedded neural prosthesis. A care-giver (CG) is also shown in Fig. 16. The embedded neural prosthesis comprises a number of simple component devices (Dₐ-Dᵢ) that self organize into a network. Component devices (Dₐ, D_{b}) include stimulatory and/or sensory electrodes inserted into the patient's brain, sensor component devices (D_{c}, D_{d}, Dₑ, D_{f}) positioned on extremities and torso, audio feedback devices (Dg) in or around the ear(s), visual devices (Dₕ), i.e., goggles, to provide an augmented visual reality, EEG sensor(s) (Dᵢ) positioned on the scalp and/or forehead. Other devices, not shown on the figure, may also be integrated into the network, such as chemical, electrophysiological or temperature sensors, and therapeutic devices such as drug delivery system, stimulatory or inhibitory electrodes inserted into the spinal cord or muscles, etc. The sensory components of the network measure certain parameters of the patient's body and physiology. This data is then analyzed within the network, with the component devices providing computational resources and communication bandwidth in a distributed fashion. Computational resources therefore may involve one, several or all of the component devices in the network. The results of the analysis are then translated into therapeutic instructions within the network. The therapeutic instructions may be derived by e.g. therapeutic component devices or by sensory component devices, or by a combination thereof. Ultimately therapeutic instructions are delivered to therapeutic component devices. These devices will then adjust their operation in order to deliver a therapeutic response prescribed in the therapeutic instructions. After executing the therapeutic action, such as delivering a stimulatory impulse through a brain electrode, delivering audio feedback (e.g. Delayed Audio Feedback), transmitting visual clues though the augmented reality visual devices, delivering a drug, etc. the therapeutic component devices report back to other component devices and inform them that the prescribed therapeutic action has been undertaken. Such measure-compute-react when necessary-report cycles are then repeated continuously.

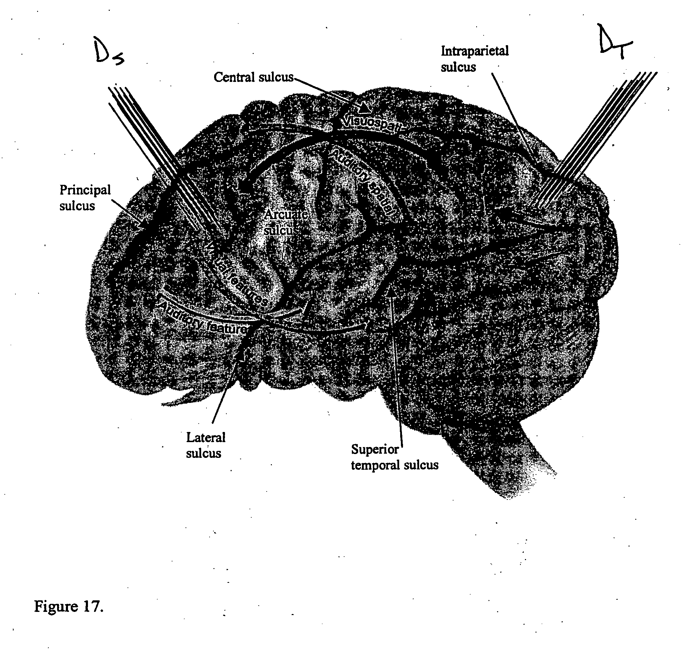

Figure 17 illustrates generally, for example, an array of sensing component devices (Dₛ) and an array of therapeutic, i.e., stimulating, electrode component devices (Dₜ) implanted in the brain of a patient. Similar such arrays can be implanted into the motor or sensory cortex, basal ganglia, etc. The devices comprising these arrays can monitor the activity of the brain or collaborate with other sensory or therapeutic devices elsewhere in, on or around the patient body to influence brain, or other, activity to the benefit of the patient.

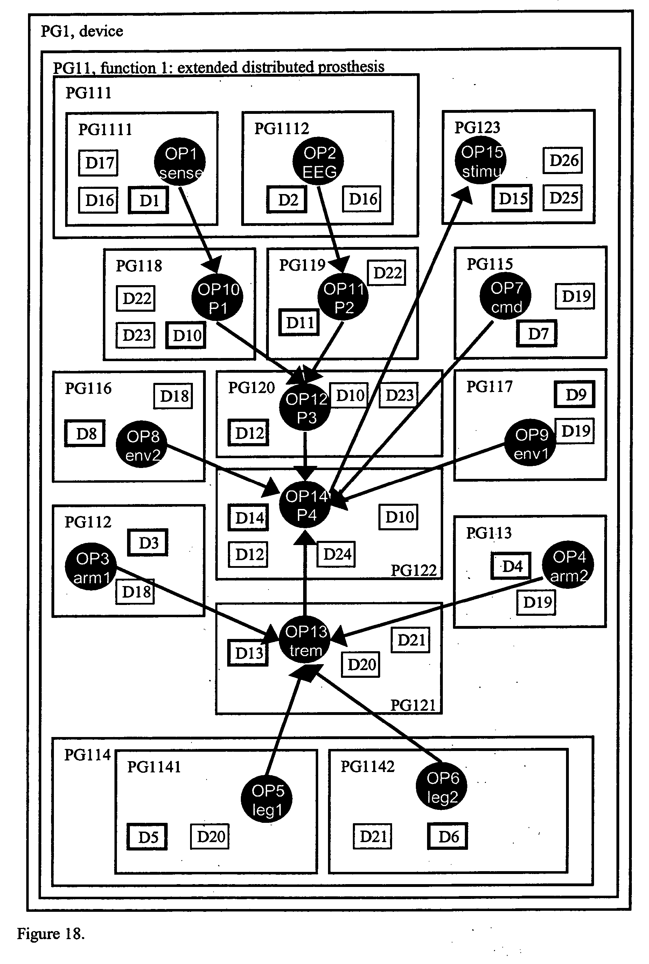

Figure 18 illustrates a network of component devices designed to receive direct patient or care-giver input and also to sense environmental parameters to deliver a therapeutic effect according to the systems and methods of the invention. Figure 18 illustrates, for example, a series of component devices (D1-D26) strategically located in, on or about the patient, or some combination thereof. The data collected by the various devices may be physiological factors such as chemical or electrical production or activity in the targeted areas whereat the devices are located. Such physiological factors may be, for example, body temperature, skin impedance, sweating, breathing rates, volumes or patterns, blood flow conditions, heart rate, etc. The data collected by the various devices is then analyzed and communicated to those of the devices having therapeutic capabilities in order to initiate and deliver an appropriate therapeutic effect to the patient based thereon. The network of component devices in the example neural prosthesis of Fig. 18 further senses environmental parameters, and analyzes and communicates the sensed environmental parameters to one or more of the therapeutic component devices to deliver the desired therapeutic effect to the patient. The therapeutic effect may be, for example, stimulating additional chemical or electrical activity in the brain, or suppressing such chemical or electrical activity in the brain, or affecting the brain through the sensory organs by means of sensory inputs.

Both sensing and therapeutic functions can be accomplished by individual component devices or various levels of peer groups in the network. Sensing physiological and environmental parameters and adjusting the performance of the network of component devices on the basis thereof provides a more refined medical device. Appropriate therapeutic action and a better integration of the patient into his environment is more likely achieved as a result. The organization of the component devices into various hierarchies within the network helps adjust the medical device to changes in patient status (e.g. progression of disease requiring more frequent sensing, computation and therapeutic action), changes in sensing needs (e.g. frequency of measurements), computational needs and changes in the availability of component devices as brought about by component device failures and the introduction of new component devices. The network's ability to self-organize and adapt to changing patient and environmental parameters, as well as the availability of its internal resources, contributes to the robustness of the medical device. The actual hierarchy, peer group composition, communication pathways, computing and therapeutic task allocation, etc. at any point in time, ideally represents the ideal network organization under the circumstances.

Referring still to Figure 18, the neural prosthesis shown therein is further designed to receive direct patient, or care-giver, input via component devices D7, D19 and operation OP7, for example, in order to deliver an appropriate therapeutic effect according to the systems and methods of the invention. The component devices D7 and D19 thus represent one or more input devices provided in, on or around the patient for manipulation by the patient or the patient care-giver as desired. For example, either or both of the direct input devices D7 and D 19 may be a timer that controls when, or how long, an input is provided to one or more of the other sensing devices or therapeutic devices. Operation of the input devices D7 and D19 is thus directly controlled by the patient or the care-giver, and may be independent of physiological parameters otherwise sensed, analyzed or communicated among the various other component devices within the network. The operation of the input devices D7 and D19 may also be controlled by the patient or care-giver based upon physiologic parameters as well. In either case, the direct input of the patient or care-giver to the network of devices via the one or more input devices D7 and D19, for example, helps the neural prosthesis deliver a therapeutic effect to the patient as desired based upon the physiological and environmental parameters, as described above, and further based upon direct patient or care-giver input. In accordance with concepts of adaptation and dynamic reorganization of component device hierarchy described in previous paragraphs, the integration of commands from input devices will affect the actual organization of component device for optimal resource and task organization and allocation.

By way of example only, wherein the artisan will appreciate that a neural prosthesis according to the systems and methods described herein may be comprised of component devices other than as shown in Fig. 18, a network of devices as shown in Figure 18 may be configured to function as follows: Operation OP1 senses brain activity through electrode arrays, operation OP2 senses EEG signals on the scalp. Operations OP3-6 senses the tremor of the limbs, and the data is preprocessed in operation OP 13. Operation OP7 receives commands from the patient or caregivers. Operations OP8-9 senses some environmental parameters, e.g. humidity, temperature. Operations OP10-12 preprocess the sensed electrical brain signals, and operation OP 14 calculates the required therapeutic effect based on the received preprocessed data, sensed environmental parameters and commands. Finally operation OP 15 delivers the required therapeutic effect, for example through an electrode array in the brain. Multiple devices execute each operation, which provides a robust behavior for the entire device.

In the example neural prosthesis shown in Fig. 18, device D1 has sensory electrode arrays and knows from its previous experiences that D2 can organize the required EEG measurement, therefore creates PG111 for OP1 and OP2 and PG1111 for OP1 and becomes the manager of that peer group. Also, D1 gives the remainder task to D2 to create PG1112 for OP2 and to organize the EEG measurement. D3-D5 accepts the limbs' tremor measurements and creates peer groups PG112-114. D5 creates PG114 for both legs' tremor measurements, because from its previous experiences it knows that D6 reliably organizes the other limb's tremor measurement. Therefore PG1141 and PG1142 are created by D5 and D6 for each limb's measurement. Then devices D7-D15 creates peer groups PG115-123 for the remaining operations: command input, environmental sensing, calculations and therapeutic effect. Then some additional devices join the peer groups to provide enough sensory information, computational capacity, and therapeutic effect. One device may belong to multiple peer groups as its capabilities allows according to the systems and methods of the invention.

## Claims

1. An embedded neural prosthesis, comprising:
two or more component devices comprising a network of devices, each device having one or more functions as a sensor, communicator, computer, data distributor, energy source, or therapeutic effector, and self-organizing into a hierarchy of various levels of devices within the network,
the devices being implanted in the human body, placed on the human body, placed in the environment within which the human body is located, or distributed in any combination thereof,
at least one of a direct communication link between the devices via cryptography and communication protocols or an indirect communication link between the devices via intermediate devices,
each of the devices containing data, algorithms and protocols to perform at least one of the following functions:
- sense physiologic parameters
- process data distributed in the system
- exchange, modify, reconfigure data, algorithms, and protocols in the prosthesis
- autonomously allocate data storage, computational, communication, energy supply, sensory and therapeutic tasks among the devices in the prosthesis,
wherein the therapeutic effector delivers an intended therapeutic effect upon completion of tasks or functions of underlying levels of devices of the network.

2. The prosthesis of claim 1, wherein the intended therapeutic effect is any of mechanical assistance, electrical stimulation, chemical stimulation, actuation, drug delivery and suppression.

3. A neural prosthesis comprising:
a hierarchical network of various levels of self-organizing devices provided in, on or about a patient and having anonymous and accountable communication capabilities, each device further having at least one task allocated thereto, the network providing a therapeutic effect that repairs, replaces or restores neural functions upon completion of the allocated tasks at the various levels of the self-organizing devices.

4. The prosthesis of claim 3, wherein the tasks are any of sensing physiological data, computing data, distributing data, communicating, managing energy, task allocation and scheduling, and delivering a therapeutic effect.

5. The prosthesis of claim 4, wherein the communication capabilities include direct symmetric and asymmetric cryptography between devices, indirect communication via intermediate devices, and pipeline protocols.

6. An embedded neural prosthesis comprising:
a network for delivering a therapeutic effect to a patient, the network comprised of self-organizing component devices arranged hierarchically at various levels of peer groups to form one or more overall Peer Group, each overall Peer Group having a task or function to perform upon completion of underlying tasks or functions of the various levels of peer groups of the component devices comprising a respective Peer Group, such that completion of the tasks of functions of each Peer Group results in the network delivering the therapeutic effect that repairs, replaces or restores neural functions;
in-range communication links between those of the component devices within a pre-determined communication range; and
out-of-range communication links between those component devices beyond the pre-determined communication range.

7. The prosthesis of claim 6, wherein the therapeutic effect is any of mechanical assistance, electrical stimulation, chemical stimulation, actuation, drug delivery and suppression.

8. The prosthesis of claim 7, wherein each of the component devices includes data, algorithms or protocols enabling at least one task or function of sensing physiological data, computing data, distributing data, communication, task allocation and scheduling, and delivering of the therapeutic effect.

9. The prosthesis of claim 8, wherein the in-range communication links further comprise symmetric and asymmetric cryptography.

10. The prosthesis of claim 9, wherein the asymmetric cryptography establishes trust between component devices, and the symmetric cryptography authorizes communication between trusted ones of the component devices.

11. The prosthesis of claim 9, wherein the out-of-range communication includes communication via intermediate component devices and pipeline protocols.

12. The prosthesis of claim 11, wherein the various levels of hierarchically arranged component devices execute prioritized tasks or functions and update relationships between component devices to accommodate delivery of the therapeutic effect by the network upon completion of underlying tasks and functions.

13. The prosthesis of claim 12, wherein each of the in-range communication links further comprise a channel, wherein communications between two or more of the component devices are scheduled to avoid communication interference by assigning the communications between the in-range component devices to a same channel.

14. The prosthesis of claim 13, wherein task allocation among the component devices is prioritized according to the efficiency of the component device to which a respective task is allocated.

15. The prosthesis of claim 6, further comprising a series of component devices within a respective Peer Group.

16. The prosthesis of claim 15, further comprising at least one common task or function between at least two component devices within a Peer Group.

17. The prosthesis of claim 16, wherein a Peer Group performs its task or function upon completion of the tasks or functions of the various underlying levels of peer groups of component devices comprising the Peer Group.

18. The prosthesis of claim 6, further comprising one-time tasks or functions such that the various levels of peer groups of component devices authorize a succeeding level or component device to perform its task or function upon completion of a preceding one of the one-time tasks or functions.

19. The prosthesis of claim 6, further comprising repeatable tasks or functions such that the various levels of the component devices include pipeline communications between some or all of the component devices to permit ongoing processing of one of the repeatable tasks or functions even as another of the repeatable tasks or functions is initiated.

20. The prosthesis of claim 19, wherein the network re-organizes the various levels of the component devices within a respective peer group according to changes in the component devices in the respective peer group or according to changes in the task or function requirements.

21. The prosthesis of claim 20, wherein the various levels of the component devices update one another upon completion of a task or function, upon performance failure of any of the component devices, or upon addition or deletion of component devices to the network.

22. The prosthesis of claim 6, wherein the communication links are wireless.

23. The prosthesis of claim 22, wherein the data communicated by the component devices comprises information having virtual identifiers embedded therein.

24. The prosthesis of claim 23, wherein the component devices further comprise an accountability aspect in the cryptography or other protocols used within the network.

25. The prosthesis of claim 6, further comprising a manager device from among the component devices at each respective peer group level, the manager device continuously evaluating the reliability of the component devices comprising the respective peer groups to ensure that underlying levels of tasks or functions are reliably and timely executed.

26. The prosthesis of claim 6, wherein the component devices comprising a peer group are organized and authorized to execute an intended task or function corresponding to a respective peer group upon an accepted response to an advertisement describing the intended task, function or resource requirements for performing said task or function.

27. A method of replacing, repairing or restoring neural function to a patient, comprising:
placing a network of two or more component devices in, on or about the patient, each component device performing at least one of sensing, computing data, distributing data, communicating, task allocating and scheduling, and delivery of a therapeutic effect;
hierarchically arranging the network of two or more component devices into various levels of peer groups, the various levels of peer groups and component devices comprising an overall Peer Group and each peer group level assigned a task or function to execute;
communicating the completion of underlying tasks or functions from one peer group level to a successor peer group level until all peer group levels have executed their assigned tasks or functions, thereby resulting in the overall Peer Group having delivered the intended therapeutic effect to the patient.

28. The method of claim 27, wherein the therapeutic effect is any of mechanical assistance, electrical stimulation, chemical stimulation, actuation, drug delivery and suppression that helps repair, replace or restore neural functions.

29. The method of claim 28, wherein the task or function of a respective peer group level is directly performed by component devices authorized to comprise the respective peer group.

30. The method of claim 28, wherein the task or function of a respective peer group level is decomposed into smaller tasks or functions resulting in the formation of another level of peer group comprised of additional component devices, wherein the additional peer group level's task or function is executed prior to execution of the originating respective peer group level.

31. The method of claim 29, wherein each peer group level is formed by responding to an advertisement describing the task or function to be performed by the respective peer group level such that the most reliable component devices for performing the advertised task or function comprise the peer group.

32. The method of claim 30, wherein each peer group level is formed by responding to an advertisement describing the task or function to be performed by the respective peer group level such that the most reliable component devices for performing the advertised task or function comprise the peer group.

33. The method of claim 31, further comprising designating a manager device from among the component devices comprising a respective peer group level, the manager device continuously evaluating the reliability of the component devices in said respective peer group level and changing or adding component devices thereto to maintain said reliability.

34. The method of claim 32, further comprising designating a manager device from among the component devices comprising a respective peer group level, the manager device continuously evaluating the reliability of the component devices in said respective peer group level and changing or adding component devices thereto to maintain said reliability.

35. An embedded neural prosthesis comprising:
a network of hierarchically arranged peer groups, the peer groups comprised of component devices arranged in, on or about a patient, the component devices configured to self-organize to accomplish various levels of tasks or functions.

36. The prosthesis of claim 35, wherein at least one of the component devices is a therapeutic device, at least one of the component devices is a sensory device, and at least one of the component devices is a computational device.

37. The prosthesis of claim 36, wherein each component device is configured to communicate with at least one other component device.

38. The prosthesis of claim 37, wherein the at least one therapeutic devices is configured to stimulate or depress a function of certain components of the nervous system in response to information received, analyzed and communicated thereto from the at least one sensory device and the at least one computational device.

39. The prosthesis of claim 37, further comprising a direct input component device configured to receive input directly from at least one of the patient or a caregiver.

40. The prosthesis of claim 39, wherein the direct input is in addition to physiological and environmental parameters otherwise sensed by the component devices of the prosthesis.

41. The prosthesis of claim 40, wherein the physiological parameters comprise at least one of body temperature, skin impedance, moisture, musculo-skeletal movements, blood flow, breathing patterns, and heart rate.

42. The prosthesis of claim 35, wherein the component devices are configured to perform active communication powered by onboard energy sources.

43. The prosthesis of claim 35, wherein the component devices are configured to perform passive communication that does not require onboard power.

44. The prosthesis of claim 36, wherein at least some of the component devices are configured to perform sensing, computing, therapeutic and communication functions, whereas others of the component devices are configured to perform only some of the sensing, computing, therapeutic and communication functions.

45. The prosthesis of claim 35, wherein the various levels of component devices are configured to repeatedly measure data, compute the data, communicate the computed data among the component devices, and react to the communicated data in order to provide a therapeutic effect that repairs, replaces or restores neural function.

46. The prosthesis of claim 45, wherein the component devices are configured to self-organize and adapt to changing physiological and environmental parameters and to changing availability of the component devices.

47. A method of monitoring physiological and environmental parameters and adjusting a therapeutic effect for repairing, replacing or restoring neural function in response thereto, the method comprising:
organizing a plurality of component devices in, on or about a patient;
collecting data of the physiological parameters of the patient via at least some of the component devices;
collecting data of the environmental parameters of the patient via at least some of the component devices;
computing the collected data;
communicating the computed data to the component devices having capacity to deliver a therapeutic effect;
delivering a therapeutic effect in response to the collected, computed and communicated data; and
repeating the collecting, computing, communicating and delivering steps continuously to adjust to changed physiological, environmental or component device conditions.

48. The method of claim 47, further comprising directly inputting data by at least one of the patient or a care-giver prior to the computing, communication and delivering steps.
